# EUROPEAN PATENT APPLICATION

(11) **EP 4 810 487 A1**
(43) Date of publication of application: **23.09.2026**
(21) Application number: 24907441.0
(22) Date of filing: 18.12.2024
(51) Int. Cl.: C07D 235/12, A61K 31/4184, A61P 29/00

(54) **METHOD FOR PRODUCING HETEROCYCLIC DERIVATIVE**

(30) Priority: 22.12.2023 JP 2023216945
(71) Applicant: Nippon Shinyaku Co., Ltd., Kyoto-shi Kyoto 601-8550 (JP)
(72) Inventor: HARABE, Tetsuji, Kyoto-shi, Kyoto 601-8550 (JP); SATAKE, Nobuya, Kyoto-shi, Kyoto 601-8550 (JP); MIKANO, Yusuke, Kyoto-shi, Kyoto 601-8550 (JP); OMACHI, Shinji, Kyoto-shi, Kyoto 601-8550 (JP); OTSU, Hironori, Kyoto-shi, Kyoto 601-8550 (JP); TAKITA, Hirofumi, Kyoto-shi, Kyoto 601-8550 (JP); FUJIWARA, Toshio, Kyoto-shi, Kyoto 601-8550 (JP); MIYAGI, Kotaro, Kyoto-shi, Kyoto 601-8550 (JP)
(74) Representative: Hoffmann Eitle
(86) International application number: PCT/JP2024/044722
(87) International publication number: WO 2025/135056

(57) **Abstract**

Disclosed is a method for producing a p-toluenesulfonate salt of a compound represented by formula (I), the method including a step for converting an N,N-dimethylacetamide solvate of a compound represented by formula (I) into a p-toluenesulfonate salt of a compound represented by formula (I).

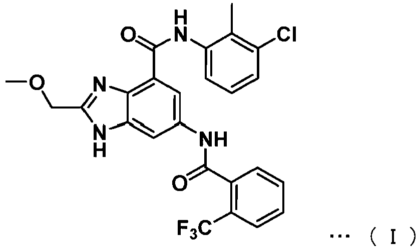

## Description

### TECHNICAL FIELD

The present disclosure relates to a production method for a heterocyclic derivative.

### BACKGROUND ART

Prostaglandins (PGs) are produced in large amounts at a site of inflammation and are involved in the development of inflammation. Among PGs, in particular, prostaglandin E2 (PGE2) induces fever, hyperalgesia, etc., as an inflammation-inducing substance in acute inflammation and chronic inflammation. PGE2 is known to be synthesized by PGE2 synthase (PGES), and PGES is responsible for the final stage of the synthesis pathway of PGE2, which is an inflammatory mediator. It has been found that there are three subtypes of PGES. Among them, like cyclooxygenase-2 (COX2), membrane-bound prostaglandin E synthase-1 (mPGES-1) is mainly induced during inflammation, and is deeply involved in PGE2 production in inflammatory lesions. Examples of active ingredients of therapeutic agents for inflammatory diseases focusing on such mechanisms include mPGES-1 inhibitors. mPGES-1 inhibitors inhibit only COX-2-dependent PGE2 production, and thus are considered to be able to reduce various side effects compared to NSAIDs and COX-2 inhibitors.

As an mPGES-1 inhibitor, PATENT DOCUMENT 1 describes N-(3-chloro-2-methylphenyl)-2-(methoxymethyl)-6-({[2-(trifluoromethyl)phenyl]carbonyl}amino)-1H-benzimidazole-4-carboxamide (hereinafter also referred to as "compound A") represented by the following formula (I).

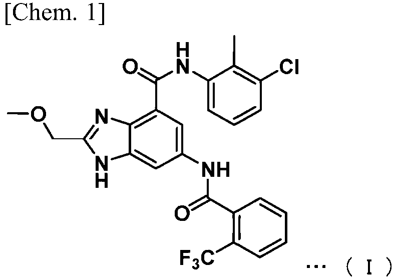

### CITATION LIST

### [PATENT DOCUMENT]

[PATENT DOCUMENT 1] WO2013/024898 A1

### SUMMARY OF THE INVENTION

### PROBLEMS TO BE SOLVED BY THE INVENTION

The production method for the compound A according to PATENT DOCUMENT 1 proceeds through a synthetic intermediate represented by the following formula (XI) (hereinafter also referred to as "compound C"), but the compound C has been found to have a high possibility of having mutagenicity (genotoxicity) by evaluation using multiple In Silico test software programs capable of predicting mutagenicity (genotoxicity).

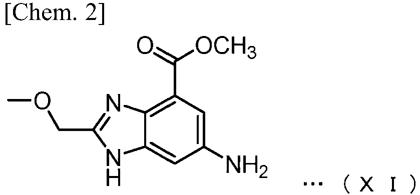

Then, if the compound A is to be produced by the method described in PATENT DOCUMENT 1, the compound C is isolated as a powder in the process, so that there has been a problem that it is necessary to perform worker protection and environmental countermeasures (containment). On the other hand, if the compound A can be produced by a synthetic route in which a powder of the compound C is not isolated, it is considered that the risk of mutagenicity due to scattering or the like of the compound C isolated as a powder can be avoided and the cost in terms of time and money for worker protection and environmental countermeasures (containment) can be reduced.

An object of the present disclosure is to provide a production method for a heterocyclic derivative. Also, an object of the present disclosure is to provide a production method for a p-toluenesulfonate of the compound A.

### SOLUTION TO THE PROBLEMS

The present inventors have found a method for producing a p-toluenesulfonate of the compound A without isolating a powder of the compound C. Furthermore, the present inventors have found that with this production method, it is possible to remove an impurity that is generated in the production process and that is required to be controlled in the order of ppm, and thus have completed the present disclosure.

The present disclosure is directed to, for example, the following.
[1] A production method for a p-toluenesulfonate of a compound represented by the following formula (I): 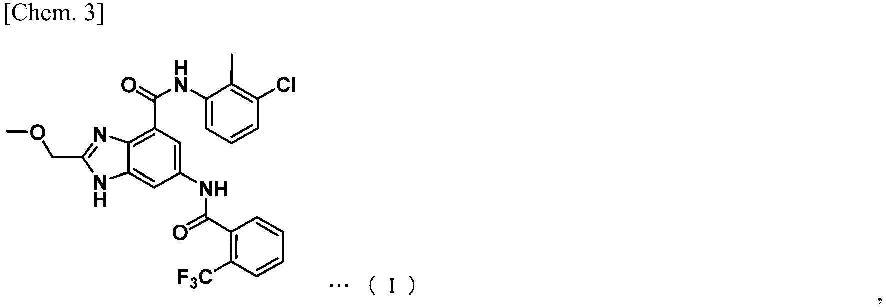 including a step of converting an N,N-dimethylacetamide solvate of the compound represented by the formula (I) into the p-toluenesulfonate of the compound represented by the formula (I).
[2] The production method according to [1], further including a step of converting a compound represented by the following formula (II): 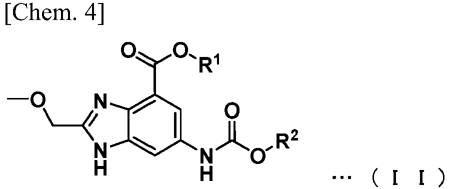 (wherein R¹ represents a protecting group for a carboxyl group or H, and R² represents a group that combines with -O-C(=O)- to form a protecting group for an aniline amino group) into an N,N-dimethylacetamide solvate of a compound represented by the following formula (I): 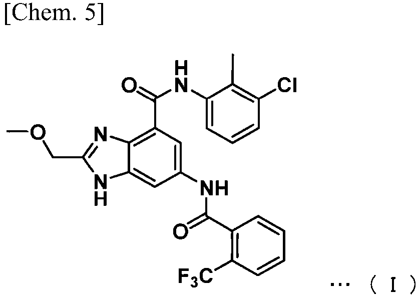
[3] The production method according to [2], wherein the step of converting the compound represented by the formula (II) into the compound represented by the formula (I) includes hydrolyzing the compound represented by the formula (II) in the presence of a base to obtain a compound represented by the following formula (III): 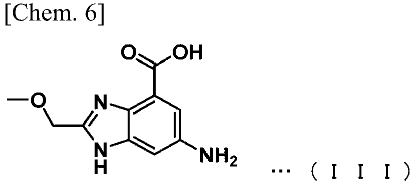
[4] The production method according to [2] or [3], wherein the step of converting the compound represented by the formula (II) into the compound represented by the formula (I) includes converting a compound represented by the following formula (III): 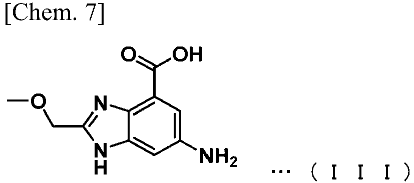 into a compound represented by the following formula (VIII): 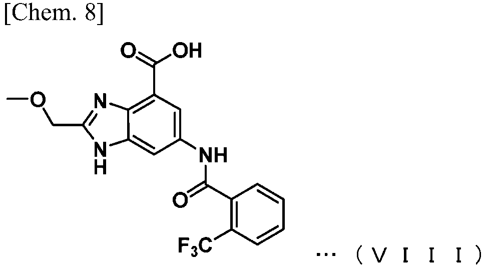
[5] The production method according to any one of [2] to [4], wherein the step of converting the compound represented by the formula (II) into the compound represented by the formula (I) includes converting a compound represented by the following formula (VIII): 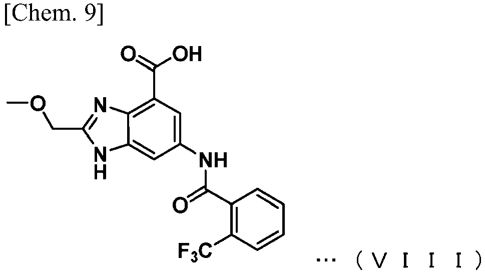 into an N,N-dimethylacetamide solvate of a compound represented by the following formula (I): 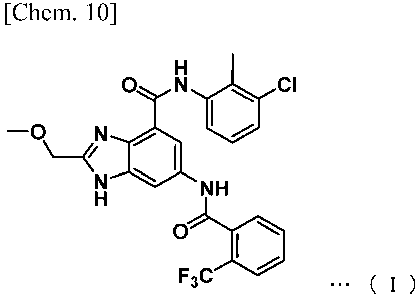
[6] The production method according to any one of [2] to [5], wherein R¹ and R² in the formula (II) each independently represent C₁-C₆ alkyl.
[7] The production method according to any one of [2] to [6], wherein R¹ and R² in the formula (II) are each methyl.
[8] The production method according to any one of [1] to [7], further including a step of obtaining the compound represented by the formula (II), including bringing a compound represented by the following formula (IV): 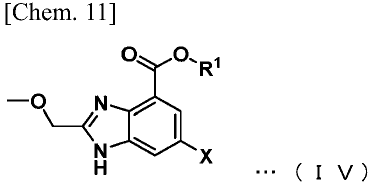
   (wherein R¹ is the same as R¹ in the formula (II), and X represents Cl, Br, I, or OTf)
   or its protected product into contact with a compound represented by the following formula (V): 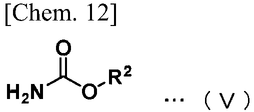
   (wherein R² is the same as R² in the formula (II))
   to obtain the compound represented by the formula (II) or its protected product.
[9] The production method according to [8], wherein X in the formula (IV) is Cl, Br, or I.
[10] The production method according to [8] or [9], wherein X in the formula (IV) is Br.
[11] The production method according to any one of [8] to [10], wherein bringing the compound represented by the formula (IV) or its protected product into contact with the compound represented by the formula (V) in the step of obtaining the compound represented by the formula (II) is performed in the presence of a palladium catalyst.
[12] The production method according to [11], wherein the palladium catalyst is a palladium catalyst containing zero-valent palladium.
[13] The production method according to [11] or [12], wherein the palladium catalyst is tris(dibenzylideneacetone)dipalladium(0).
[14] The production method according to any one of [11] to [13], wherein bringing the compound represented by the formula (IV) or its protected product into contact with the compound represented by the formula (V) in the step of obtaining the compound represented by the formula (II) is performed in the presence of a ligand.
[15] The production method according to [14], wherein the ligand is 2-dicyclohexylphosphino-2',4',6'-triisopropylbiphenyl.
[16] The production method according to any one of [8] to [15], wherein the compound represented by the formula (IV) or its protected product is a compound represented by the following formula (IX): 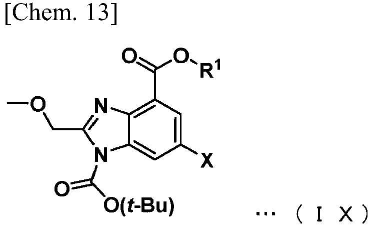 (wherein R¹ is the same as R¹ in the formula (II), and X is the same as X in the formula (IV)).
[17] The production method according to any one of [8] to [16], wherein the step of obtaining the compound represented by the formula (II), including bringing the compound represented by the formula (IV) or its protected product into contact with the compound represented by the formula (V), includes, in the following order,
   converting the compound represented by the formula (IV) into the compound represented by the formula (IX),
   bringing the compound represented by the formula (IX) into contact with the compound represented by the formula (V) to obtain a compound represented by the following formula (X): 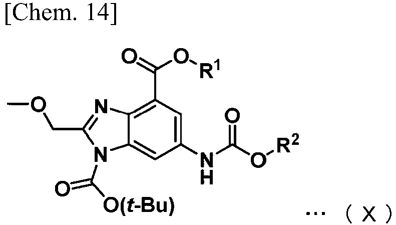 (wherein R¹ is the same as R¹ in the formula (II), and R² is the same as R² in the formula (V)), and
   hydrolyzing the compound represented by the formula (X) in the presence of an acid to obtain the compound represented by the formula (II).
[18] The production method according to any one of [1] to [17], further including a step of bringing a compound represented by the following formula (VI): 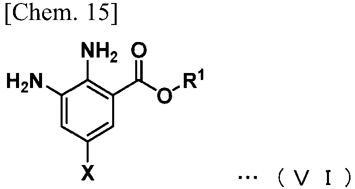 (wherein R¹ is the same as R¹ in the formula (II), and X is the same as X in the formula (IV)) into contact with methoxyacetic acid to obtain the compound represented by the formula (IV).
[19] The production method according to any one of [1] to [18], further including a step of purifying the N,N-dimethylacetamide solvate of the compound represented by the formula (I), by recrystallization.
[20] The production method according to [19], wherein recrystallization of the N,N-dimethylacetamide solvate of the compound represented by the formula (I) is performed using a mixed solvent of N,N-dimethylacetamide and water.
[21] The production method according to [19] or [20], wherein recrystallization of the N,N-dimethylacetamide solvate of the compound represented by the formula (I) is performed using a mixed solvent of N,N-dimethylacetamide and water in which a content of N,N-dimethylacetamide is 75 vol% or more.
[22] The production method according to [1] to [21], including a step of purifying the p-toluenesulfonate of the compound represented by the formula (I) by bringing the p-toluenesulfonate into contact with activated carbon after the step of converting the N,N-dimethylacetamide solvate of the compound represented by the formula (I) into the p-toluenesulfonate of the compound represented by the formula (I), and a step of purifying the p-toluenesulfonate of the compound represented by the formula (I) after being brought into contact with the activated carbon, by recrystallization.
[23] The production method according to [22], wherein a mass of the activated carbon to be brought into contact with the p-toluenesulfonate of the compound represented by the formula (I) is 0.04 times or more a mass of the p-toluenesulfonate of the compound represented by the formula (I).
[24] A production method for a p-toluenesulfonate of a compound represented by the following formula (I): 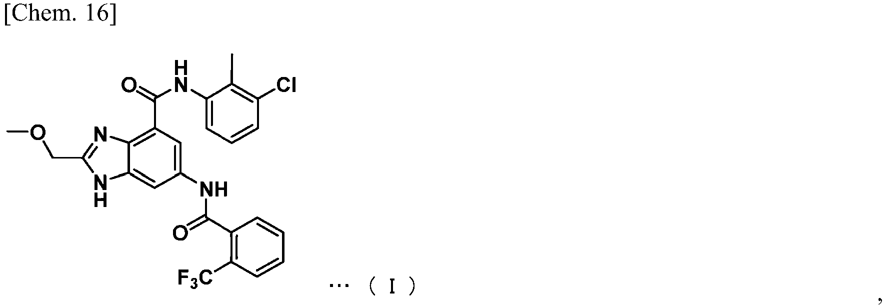
   including, in the following order:
      a step of obtaining the compound represented by the formula (II), including bringing a compound represented by the following formula (IV): 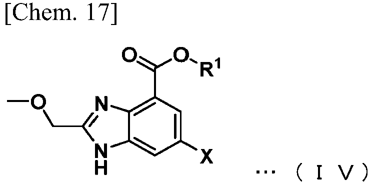
   (wherein R¹ represents a protecting group for a carboxyl group or H, and X represents Cl, Br, I, or OTf)
   or its protected product into contact with a compound represented by the following formula (V): 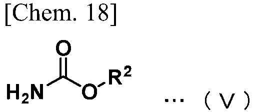
   (wherein R² represents a group that combines with -O-C(=O)- to form a protecting group for an aniline amino group)
   to obtain a compound represented by the following formula (II): 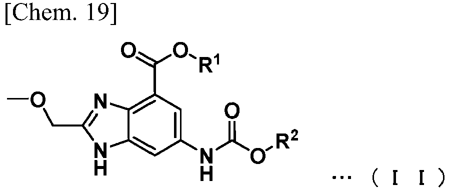
   (wherein R¹ is the same as R¹ in the formula (IV), and R² is the same as R² in the above formula (V))
   or its protected product;
      a step of converting the compound represented by the formula (II) into an N,N-dimethylacetamide solvate of the compound represented by the formula (I), including
         hydrolyzing the compound represented by the formula (II) in the presence of a base to obtain a compound represented by the following formula (III): 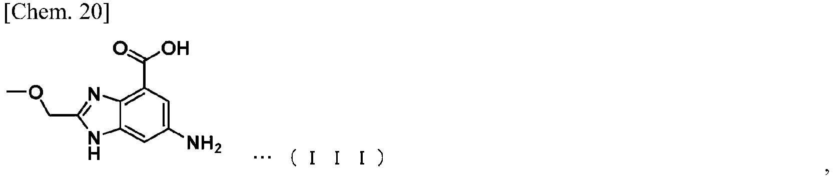
         converting the compound represented by the formula (III) into a compound represented by the following formula (VIII): 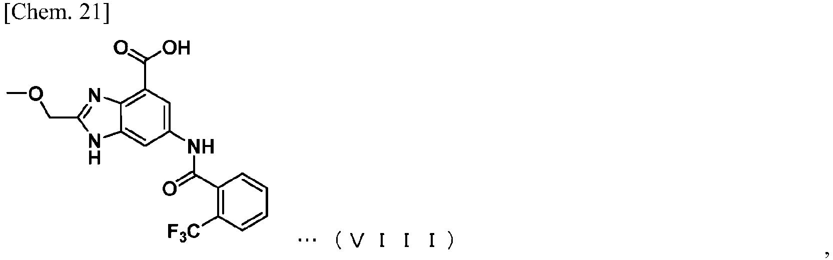 and
         converting the compound represented by the formula (VIII) into the N,N-dimethylacetamide solvate of the compound represented by the formula (I); and
      a step of converting the N,N-dimethylacetamide solvate of the compound represented by the formula (I) into a p-toluenesulfonate of the compound represented by the formula (I).
[25] A production method for a p-toluenesulfonate of a compound represented by the following formula (I): 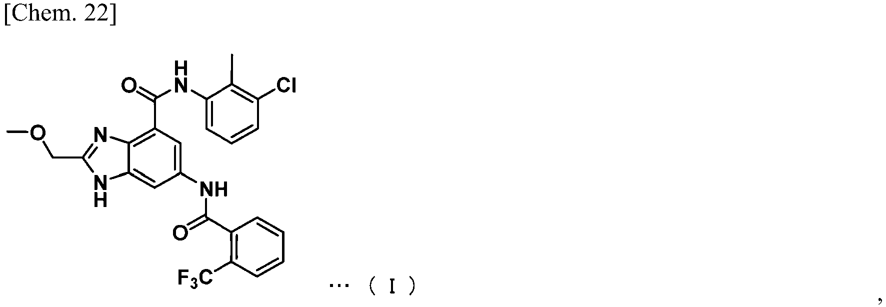
   including, in the following order:
      a step of bringing a compound represented by the following formula (VI): 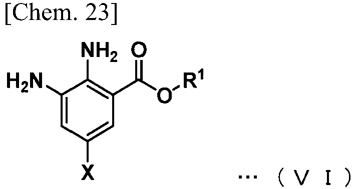
   (wherein R¹ represents a protecting group for a carboxyl group or H, and X represents Cl, Br, I, or OTf)
   into contact with methoxyacetic acid to obtain a compound represented by the following formula (IV): 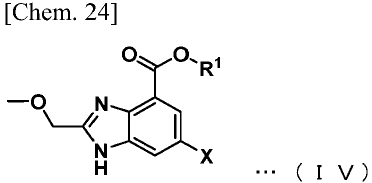
   (wherein R¹ and X are the same as R¹ and X in the formula (VI), respectively);
      a step of converting the compound represented by the formula (IV) into a compound represented by the following formula (II): 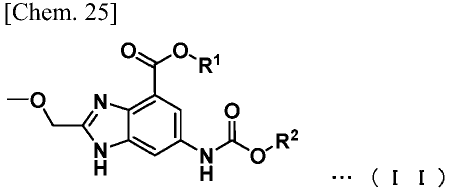
   (wherein R¹ is the same as R¹ in the formula (VI), and R² represents a group that combines with - O-C(=O)- to form a protecting group for an aniline amino group),
   including, in the following order,
      converting the compound represented by the formula (IV) into a compound represented by the following formula (IX): 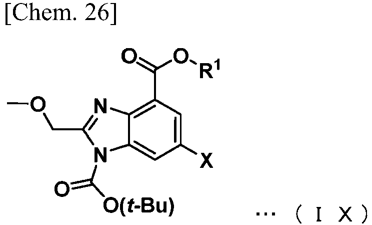
   (wherein R¹ and X are the same as R¹ and X in the formula (VI), respectively),
      bringing the compound represented by the formula (IX) into contact with a compound represented by the following formula (V): 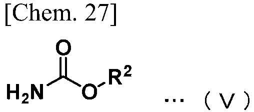
   (wherein R² is the same as R² in the formula (II))
   to obtain a compound represented by the following formula (X): 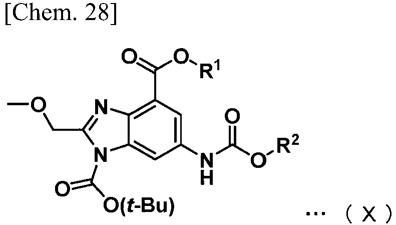
   (wherein R¹ is the same as R¹ in the formula (VI), and R² is the same as R² in the formula (II)), and
      hydrolyzing the compound represented by the formula (X) in the presence of an acid to obtain the compound represented by the formula (II);
      a step of converting the compound represented by the formula (II) into an N,N-dimethylacetamide solvate of the compound represented by the formula (I), including
         hydrolyzing the compound represented by the formula (II) in the presence of a base to obtain a compound represented by the following formula (III): 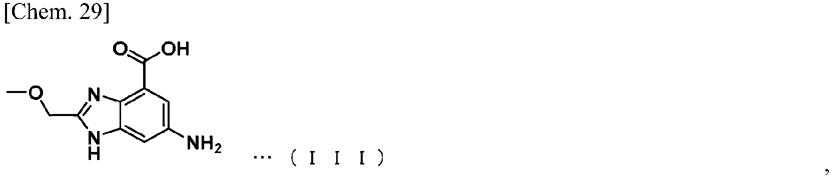
         converting the compound represented by the formula (III) into a compound represented by the following formula (VIII): 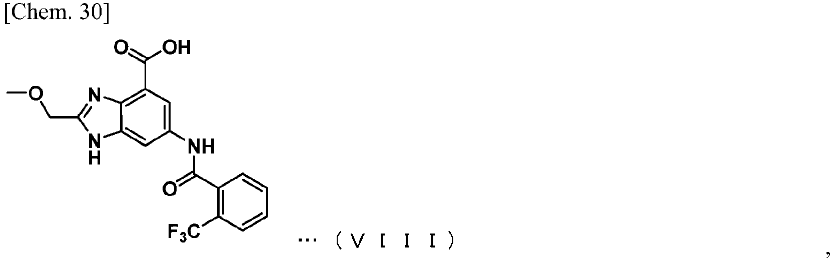 and
         converting the compound represented by the formula (VIII) into the N,N-dimethylacetamide solvate of the compound represented by the formula (I);
      a step of purifying the N,N-dimethylacetamide solvate of the compound represented by the formula (I), by recrystallization;
      a step of converting the N,N-dimethylacetamide solvate of the compound represented by the formula (I) into a p-toluenesulfonate of the compound represented by the formula (I);
      a step of purifying the p-toluenesulfonate of the compound represented by the formula (I) by bringing the p-toluenesulfonate into contact with activated carbon; and
      a step of purifying the p-toluenesulfonate of the compound represented by the formula (I) after being brought into contact with the activated carbon, by recrystallization.
[26] The production method according to [24] or [25], wherein R¹ and R² are each C₁-C₆ alkyl and preferably methyl, and X is Cl, Br, or I and preferably Br.
[27] The production method according to [25], wherein
   R¹ and R² are each C₁-C₆ alkyl and preferably methyl,
   X is Cl, Br, or I and preferably Br,
   bringing the compound represented by the formula (IV) or its protected product into contact with the compound represented by the formula (V) is performed in the presence of a palladium catalyst,
   recrystallization of the N,N-dimethylacetamide solvate of the compound represented by the formula (I) is performed using a mixed solvent of N,N-dimethylacetamide and water, and is preferably performed using a mixed solvent of N,N-dimethylacetamide and water in which a content of N,N-dimethylacetamide is 75 vol% or more, and
   a mass of the activated carbon to be brought into contact with the p-toluenesulfonate of the compound represented by the formula (I) is 0.04 times or more and preferably 0.08 times or more a mass of the p-toluenesulfonate of the compound represented by the formula (I).
[28] The production method according to any one of [24] to [27], wherein bringing the compound represented by the formula (IV) or its protected product into contact with the compound represented by the formula (V) is performed in the presence of a ligand and a palladium catalyst containing zero-valent palladium, and is preferably performed in the presence of tris(dibenzylideneacetone)dipalladium(0) and 2-dicyclohexylphosphino-2',4',6'-triisopropylbiphenyl.
[29] An N,N-dimethylacetamide solvate of a compound represented by the following formula (I): 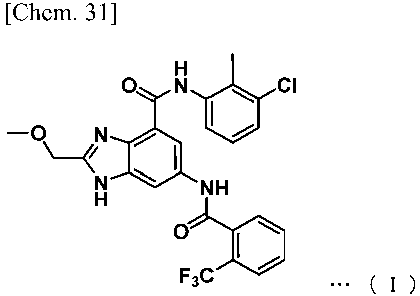
[30] A composition containing an N,N-dimethylacetamide solvate of a compound represented by the following formula (I): 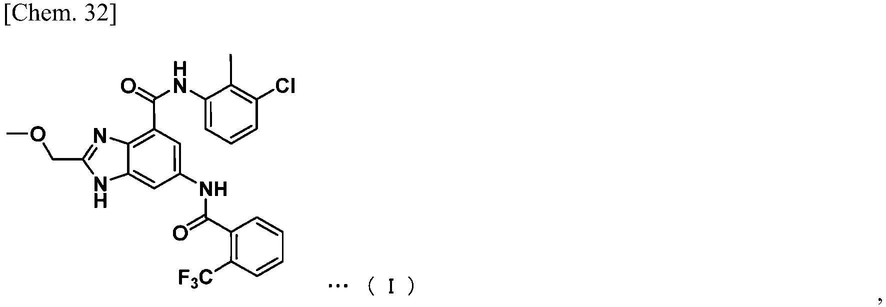
   wherein a content of the N,N-dimethylacetamide solvate of the compound represented by the formula (I) is 95 mass% or more of the entire composition, and a content of a compound represented by the following formula (VII): 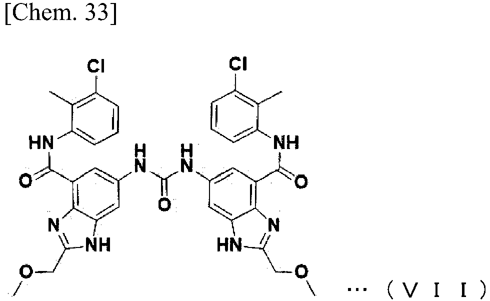
   is 1800×10⁻⁴ mass% or less and preferably 200×10⁻⁴ mass% or less of the entire composition in terms of free form.
[31] A composition containing an N,N-dimethylacetamide solvate of a compound represented by the following formula (I): 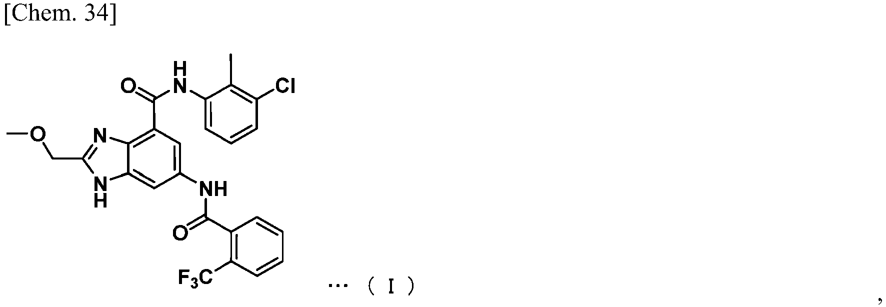 and
   a compound represented by the following formula (VII): 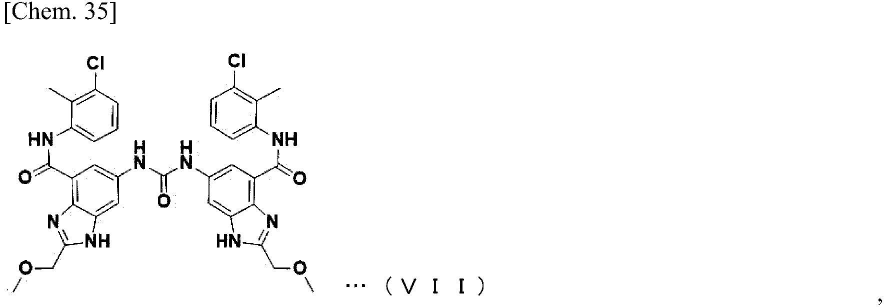
   wherein a content of the N,N-dimethylacetamide solvate of the compound represented by the formula (I) is 95 mass% or more of the entire composition, and a content of the compound represented by the formula (VII) is 1800×10⁻⁴ mass% or less and preferably 200×10⁻⁴ mass% or less of the entire composition in terms of free form.
[32] The salt according to [29] or the composition according to [30] or [31] recrystallized using a mixed solvent of N,N-dimethylacetamide and water.
[33] The salt according to [29] or the composition according to [30] or [31] recrystallized using a mixed solvent of N,N-dimethylacetamide and water in which a content of N,N-dimethylacetamide is 75 vol% or more.
[34] A composition containing a p-toluenesulfonate of a compound represented by the following formula (I): 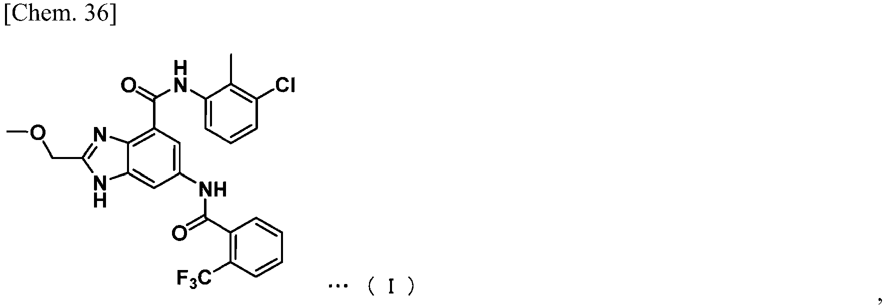
   wherein a content of the p-toluenesulfonate of the compound represented by the formula (I) is 95 mass% or more of the entire composition, and a content of a compound represented by the following formula (VII): 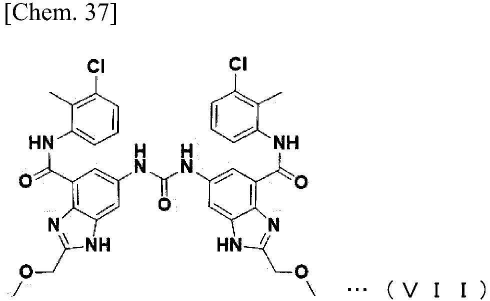
   is 100×10⁻⁴ mass% or less and preferably 50×10⁻⁴ mass% or less in terms of free form.
[35] A composition containing a p-toluenesulfonate of a compound represented by the following formula (I): 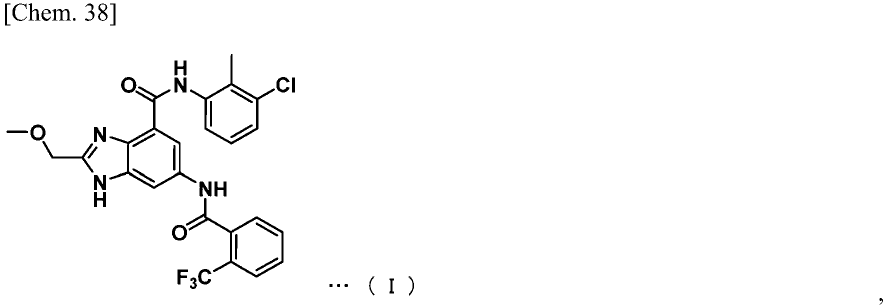 and
   a compound represented by the following formula (VII): 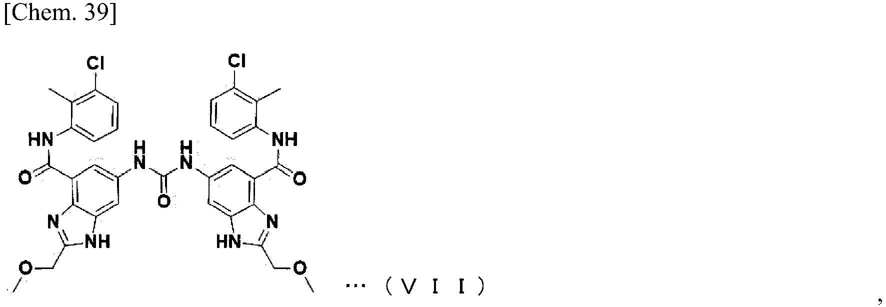
   wherein a content of the p-toluenesulfonate of the compound represented by the formula (I) is 95 mass% or more of the entire composition, and a content of the compound represented by the formula (VII) is 100×10⁻⁴ mass% or less and preferably 50×10⁻⁴ mass% or less in terms of free form.
[36] The composition according to [34] or [35] purified by recrystallization after being purified by being brought into contact with activated carbon.
[37] The composition according to [34] or [35] purified by recrystallization after being purified by being brought into contact with activated carbon whose mass is 0.04 times or more and preferably 0.08 times or more a mass of the p-toluenesulfonate of the compound represented by the formula (I).
[38] The salt or composition according to any one of [29] to [37] produced by the production method according to any one of [1] to [28] or a production method including one or more of the steps thereof.
[39] A purification method for a compound represented by the following formula (I): 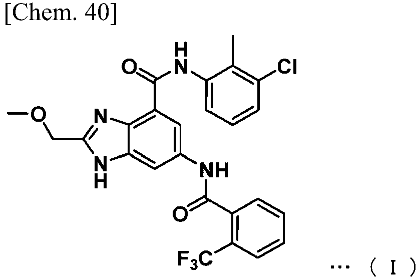 or its p-toluenesulfonate, the purification method including, in the following order:
   a step of recrystallizing an N,N-dimethylacetamide solvate of the compound represented by the formula (I), using a mixed solvent of N,N-dimethylacetamide and water in which a content of N,N-dimethylacetamide is 75 vol% or more;
   a step of bringing the p-toluenesulfonate of the compound represented by the formula (I), into contact with activated carbon whose mass is 0.04 times or more a mass of the p-toluenesulfonate of the compound represented by the formula (I); and
   a step of recrystallizing the p-toluenesulfonate of the compound represented by the formula (I) after being brought into contact with the activated carbon.

### ADVANTAGEOUS EFFECTS OF THE INVENTION

According to one embodiment of the present disclosure, it is possible to provide a production method for a heterocyclic derivative. In addition, according to one embodiment of the present disclosure, it is possible to provide a production method for the p-toluenesulfonate of the compound A.

With the production method according to one embodiment of the present disclosure, it is possible to produce the p-toluenesulfonate of the compound A or a composition containing the same without proceeding through isolation of the compound C as a powder.

With the production method according to one embodiment of the present disclosure, it is possible to produce the p-toluenesulfonate of the compound A or a composition containing the same in which the content of a compound (hereinafter also referred to as "compound B") represented by the following formula (VII): 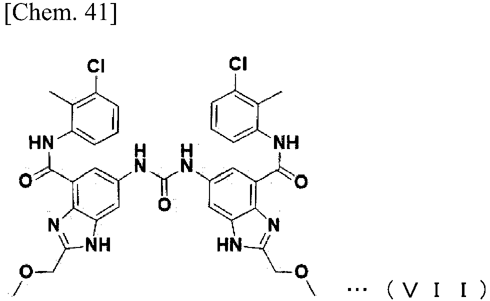 is reduced. The compound B may also be involved in mutagenicity and may be generated in the process of production, but in the production method according to one embodiment of the present disclosure, the amount of the compound B can be reduced, and in addition, the compound B can be removed with high efficiency in one embodiment.

With the production method according to one embodiment of the present disclosure, it is possible to produce the p-toluenesulfonate of the compound A or a composition containing the same in which contamination of mutagenic impurities is suppressed. With the production method according to one embodiment of the present disclosure, it is possible to produce the p-toluenesulfonate of the compound A or a composition containing the same in which the content of the compound B is reduced.

In addition, the method described in PATENT DOCUMENT 1 includes a step of nitration in concentrated sulfuric acid/concentrated nitric acid and catalytic reduction with hydrogen in order to introduce a primary amino group, so that there are concerns about explosiveness in manufacturing on an industrial scale. On the other hand, in the production method according to one embodiment of the present disclosure, a primary amino group is introduced by a step involving a mild coupling reaction, so that it is possible to more safely produce the p-toluenesulfonate of the compound A or a composition containing the same by avoiding a potentially explosive step.

According to one embodiment of the present disclosure, it is possible to provide the N,N-dimethylacetamide solvate of the compound A or a composition containing the same. By using the N,N-dimethylacetamide solvate of the compound A, it is possible to produce the p-toluenesulfonic acid of the compound A or a composition containing the same in which contamination of mutagenic impurities is suppressed. In addition, by using the N,N-dimethylacetamide solvate of the compound A, it is possible to produce the p-toluenesulfonic acid of the compound A or a composition containing the same in which the content of the compound B is reduced. That is, in one embodiment of the present disclosure, the p-toluenesulfonic acid of the compound A or a composition containing the same in which the content of the compound B is reduced is provided, and in another embodiment of the present disclosure, the N,N-dimethylacetamide solvate of the compound A or a composition containing the same in which the content of the compound B is reduced is provided.

### BRIEF DESCRIPTION OF THE DRAWINGS

[FIG. 1] FIG. 1 shows the results of X-ray powder crystal analysis of a powder obtained in step 8 of Example 1.
[FIG. 2] FIG. 2 shows the results of DSC of the powder obtained in step 8 of Example 1.
[FIG. 3] FIG. 3 shows the results of TG-DTA of the powder obtained in step 8 of Example 1.
[FIG. 4] FIG. 4 shows the results of X-ray powder crystal analysis of a powder obtained in step 5 of Example 4.
[FIG. 5] FIG. 5 shows the results of X-ray powder crystal analysis of a powder obtained in step 6 of Example 4.
[FIG. 6] FIG. 6 shows the results of thermophysical property measurement by DSC of the powder obtained in step 6 of Example 4.
[FIG. 7] FIG. 7 shows the results of X-ray powder crystal analysis of a powder obtained in step 7 of Example 4.
[FIG. 8] FIG. 8 shows the results of TG-DTA of the powder obtained in step 7 of Example 4.
[FIG. 9] FIG. 9 shows the results of X-ray powder diffraction of a powder obtained in Reference Example 1.
[FIG. 10] FIG. 10 shows the results of DSC-TGA of the powder obtained in Reference Example 1.

### DETAILED DESCRIPTION

Hereinafter, embodiments for carrying out the present disclosure will be described, but the present disclosure is not limited to the following embodiments.

In the present disclosure, unless otherwise specified, the term "compound" includes any form of the compound, including the free form of the compound, salts thereof, and solvates thereof, etc. Meanwhile, the terms "hydrochloride of the compound", "N,N-dimethylacetamide solvate of the compound", "p-toluenesulfonate of the compound", etc., refer to the stated salt or solvate of the compound and do not include the compound in other forms. More specifically, for example, "converting a compound represented by structural formula (1) into a compound represented by structural formula (2)" means converting the compound represented by structural formula (1) in any form into the compound represented by structural formula (2) in any form. For example, the phrase "hydrolyzing the compound represented by structural formula (1) to obtain an N,N-dimethylacetamide solvate of the compound represented by structural formula (2)" means hydrolyzing the compound represented by structural formula (1) in any form to obtain the compound represented by structural formula (2) in the form of an N,N-dimethylacetamide solvate. For example, the phrase "bringing a hydrochloride of the compound represented by structural formula (1) into contact with a compound represented by structural formula (3) to obtain a p-toluenesulfonate of the compound represented by structural formula (2)" means bringing the compound represented by structural formula (1) in the form of a hydrochloride into contact with the compound represented by structural formula (3) in any form to obtain the compound represented by structural formula (2) in the form of a p-toluenesulfonate. In the present disclosure, the "N,N-dimethylacetamide solvate of the compound" refers to a solvate obtained by solvating the compound with N,N-dimethylacetamide. The p-toluenesulfonate of the compound according to the present disclosure may be, for example, a salt in which the ratio between the number of molecules of the compound and the number of molecules of p-toluenesulfonic acid is 1:1. The N,N-dimethylacetamide solvate of the compound according to the present disclosure may be, for example, a solvate in which the ratio between the number of molecules of the compound and the number of molecules of the N,N-dimethylacetamide solvate is 1:1.

The chemical reactions in the present disclosure may be quenched by methods commonly used by those skilled in the art, and the products may be collected by methods commonly used by those skilled in the art, such as distillation under reduced pressure, filtration, or extraction. The products by the chemical reactions in the present disclosure may also be purified by methods commonly used by those skilled in the art, such as recrystallization or column chromatography. From the viewpoint of mass production, in one preferred embodiment, purification may be performed by recrystallization. The reactions in each step in the present disclosure may be tracked by methods commonly used by those skilled in the art, such as chromatography including reversed-phase liquid chromatography (HPLC), and the products may be analyzed by methods commonly used by those skilled in the art, such as HPLC, nuclear magnetic resonance (NMR), mass spectrometry, X-ray crystallography, DSC, and TG-DTA.

### <First aspect: Production method for p-toluenesulfonate of compound A>

One embodiment of each step of a production method for a p-toluenesulfonate of a compound (hereinafter sometimes referred to as "compound A") represented by the following formula (I): 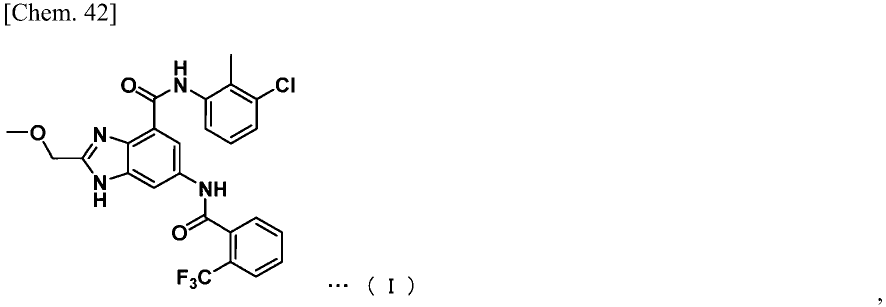 according to a first aspect of the present disclosure, will be described below.

### <First step>

A first step is a step of obtaining a compound represented by the following formula (IV): 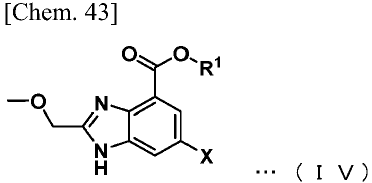 (wherein R¹ represents a protecting group for a carboxyl group or H, and X represents Cl, Br, I, or OTf).

R¹ in the above formula (IV) is a protecting group for a carboxyl group or H, and preferably a protecting group for a carboxyl group. In the present disclosure, the protecting group for a carboxyl group is not particularly limited as long as the protecting group for a carboxyl group is a group that is not removed in a second/third step described later and that can be removed in a fourth step described later without breaking at least bonds other than urethane bonds in a compound represented by the following formula (II): 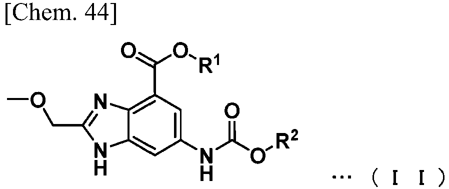 (wherein R¹ is the same as R¹ in the above formula (VI), and R² combines with -O-C(=O)- to form a protecting group for an aniline amino group) to obtain a compound in which R¹ in the above formula (II) is H. R¹ may be, for example, alkyl, cycloalkyl, alkenyl, alkoxymethyl, or aralkyl, may be C₁-C₆ alkyl, C₄-C₆ cycloalkyl, C₁-C₆ alkenyl, C₁-C₆ alkoxymethyl (C₁-C₆ alkoxylated methyl), benzyl, phenethyl, or para-methoxybenzyl in one embodiment, may be C₁-C₆ alkyl in one preferred embodiment, may be methyl, ethyl, n-propyl, isopropyl, n-butyl, t-butyl, n-pentyl, n-hexyl, cyclopentyl, cyclohexyl, vinyl, allyl, methoxymethyl, benzyl, phenethyl, or para-methoxybenzyl in another preferred embodiment, may be methyl or ethyl in a more preferred embodiment, and may be methyl in a most preferred embodiment.

X in the above formula (IV) is Cl, Br, I, or OTf (O-SO₂-CF₃), may be Cl, Br, or I in one preferred embodiment, may be Cl or Br in a more preferred embodiment, and may be Br in a most preferred embodiment.

In a most preferred embodiment, the compound represented by the above formula (IV) may be methyl 6-bromo-2-(methoxymethyl)-1H-benzimidazole-4-carboxylate represented by the following formula (IV'): 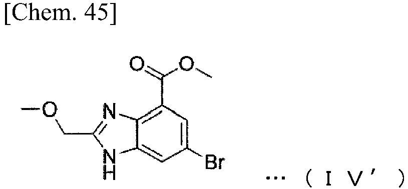 The form of the above formula (IV') obtained in the first step is not particularly limited, and may be the free form in one embodiment.

In one embodiment, the first step may be a step of bringing a compound represented by the following formula (VI): 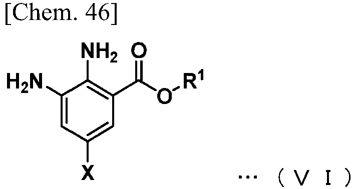
(wherein R¹ and X are the same as R¹ and X in formula (IV))
into contact with methoxyacetic acid to obtain the compound represented by the above formula (IV).

The temperature at which the compound represented by the above formula (VI) is brought into contact with methoxyacetic acid is not particularly limited as long as this temperature is a temperature at which the imidazole ring of the compound represented by the above formula (IV) is formed, and may be 50°C to 150°C, 60°C to 120°C, 70°C to 105°C, or 80°C to 95°C, for example. When the compound represented by the above formula (VI) is brought into contact with methoxyacetic acid, the contact may be achieved in a solvent. In one preferred embodiment, from the viewpoint of causing the reaction with high efficiency, the contact may be achieved by dissolving the compound represented by the above formula (VI) in methoxyacetic acid without using a solvent. When a solvent is used, a solvent in which the compound represented by the above formula (VI) and methoxyacetic acid are dissolved and whose boiling point is equal to or higher than the above temperature range is suitable as the solvent, and, for example, N,N-dimethylformamide (DMF), N,N-dimethylacetamide (DMA), etc., are suitable as the solvent.

The reaction time in the contact between the compound represented by the above formula (VI) and methoxyacetic acid is not particularly limited as long as the reaction time is a time that allows the compound represented by the above formula (IV) to be obtained, and may be 5 hours to 20 hours, for example. In addition, during this time, a reaction vessel may be purged with an inert gas such as nitrogen or argon.

The equivalent relationship between the compound represented by the above formula (VI) and methoxyacetic acid in the contact therebetween is not particularly limited as long as the compound represented by the above formula (IV) can be obtained at a high yield (e.g., a yield of 75% or more) relative to the compound represented by the above formula (VI), which is a raw material. In one embodiment, the equivalent of methoxyacetic acid may be, for example, 1 to 100, 2 to 50, or 4 to 25 equivalents per equivalent of the compound represented by the above formula (VI), and may be 10 equivalents as a specific example.

### <Second/third step>

A second/third step is a step of obtaining a compound represented by the following formula (II): 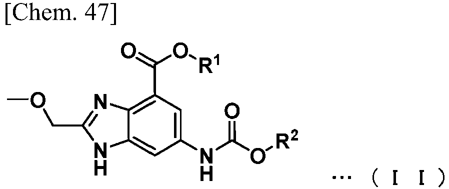
(wherein R¹ is the same as R¹ in the above formula (VI), and R² represents a group that combines with -O-C(=O)- to form a protecting group for an aniline amino group).
The second/third step includes bringing the compound represented by the above formula (IV) or its protected product into contact with a compound represented by the following formula (V): 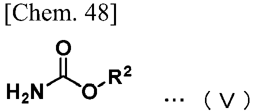
(wherein R² is the same as R² in the above formula (II))
to obtain the compound represented by the above formula (II) or its protected product.

R² in the above formula (II) represents a group that combines with -O-C(=O)- to form a protecting group for an aniline amino group. The protecting group for an aniline amino group in this case is not particularly limited as long as this protecting group is a protecting group that is not removed in the process of removing R¹ in the above formula (II) to convert R¹ to H in the fourth step described later. R² forming such a protecting group for an aniline amino group may be, for example, alkyl, cycloalkyl, alkenyl, or a hydrocarbon group having one or more aromatic rings, may be C₁-C₆ alkyl, C₄-C₆ cycloalkyl, C₁-C₆ alkenyl, benzyl, phenethyl, para-methoxybenzyl, or 9-fluorenylmethyl in one embodiment, may be C₁-C₆ alkyl in one preferred embodiment, may be methyl, ethyl, n-propyl, isopropyl, n-butyl, t-butyl, n-pentyl, n-hexyl, cyclopentyl, cyclohexyl, vinyl, allyl, benzyl, phenethyl, or 9-fluorenylmethyl in another preferred embodiment, may be methyl or ethyl in a more preferred embodiment, and may be methyl in a most preferred embodiment.

In a most preferred embodiment, the compound represented by the above formula (II) may be methyl 6-[(methoxycarbonyl)amino]-2-(methoxymethyl)-1H-benzimidazole-4-carboxylate represented by the following formula (II'): 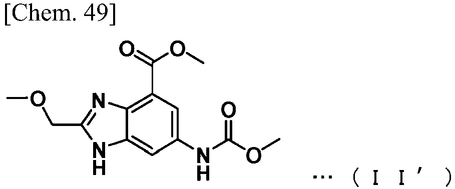 The form of the compound represented by the above formula (II') and obtained in the second/third step is not particularly limited, and in one embodiment, this form may be the form of a salt of an acid used in deprotection of a secondary amino group at 1-position described later, or may be the form of a hydrochloride, for example. In the above most preferred embodiment, the compound represented by the above formula (V) is methyl carbamate represented by the following formula (V'):

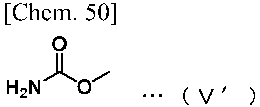

The protected product of the compound represented by the above formula (IV) means a compound represented by the following formula (IV''): 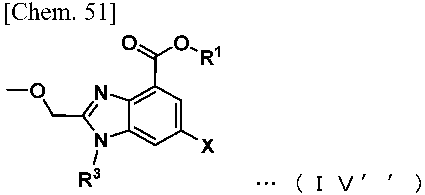 (wherein R¹ and X are the same as R¹ and X in the above formula (VI), and R³ represents a protecting group for an amino group, and represents, for example, a t-butoxycarbonyl (Boc) group, an allyloxycarbonyl (Alloc) group, a 9-fluorenylmethyloxycarbonyl (Fmoc) group, a trimethylsilyl (TMS) group, or a benzyl group).

The second/third step includes bringing the compound represented by the above formula (IV) or its protected product into contact with the compound represented by the above formula (V) to obtain the compound represented by the above formula (II) or its protected product. While not wishing to be bound by any theory, a functional group represented by X in the compound represented by the above formula (IV) or its protected product is substituted with the nitrogen atom in the terminal amide of the compound represented by the above formula (V), by Buchwald-Hartwig cross-coupling, to obtain the compound represented by the above formula (II) or its protected product.

The temperature at which the compound represented by the above formula (IV) or its protected product is brought into contact with the compound represented by the above formula (V) is not particularly limited as long as the compound represented by the above formula (II) or its protected product can be obtained, and may be 60°C to 120°C, 70°C to 110°C, 80 to 105°C, or 88°C to 97°C, for example. At this time, a solvent used for bringing the compound represented by the above formula (IV) or its protected product into contact with the compound represented by the above formula (V) is not particularly limited as long as the solvent is a solvent whose boiling point is equal to or higher than the above temperature range or that is capable of heating reflux in the above temperature range, and is a solvent that is not reactive with any of the compound represented by the above formula (IV), its protected product, and the compound represented by the above formula (V) in the reaction system. For example, toluene or 1,4-dioxane is suitable for use as the solvent, and the solvent may be toluene in one preferred embodiment. In addition, the time for bringing the compound represented by the above formula (IV) or its protected product into contact with the compound represented by the above formula (V) is not particularly limited, and may be 10 minutes to 168 hours, for example. During this time, the reaction vessel may be purged with an inert gas such as nitrogen or argon.

The equivalent relationship when bringing the compound represented by the above formula (IV) or its protected product into contact with the compound represented by the above formula (V) is not particularly limited as long as the compound represented by the above formula (II) or its protected product can be obtained at a high yield (e.g., a yield of 85% or more) relative to the compound represented by the above formula (IV) or its protected product which is a starting material. For example, the equivalent of the compound represented by the above formula (V) may be 1 to 5, 1.2 to 4, or 1.5 to 3 equivalents per equivalent of the compound represented by the above formula (IV) or its protected product, and may be 2 equivalents as a specific example.

In one embodiment, bringing the compound represented by the above formula (IV) or its protected product into contact with the compound represented by the above formula (V) is performed in the presence of a palladium catalyst. The palladium catalyst is not particularly limited as long as the compound represented by the above formula (II) or its protected product can be obtained by bringing the compound represented by the above formula (IV) or its protected product into contact with the compound represented by the above formula (V), and the palladium catalyst may be, for example, a palladium catalyst containing zero-valent palladium (Pd(0)) or a palladium catalyst containing divalent palladium (Pd(2)), and may be a palladium catalyst containing zero-valent palladium (Pd(0)) in one embodiment. Specific examples of the palladium catalyst include tris(dibenzylideneacetone)dipalladium(0) (Pd₂(dba)₃), bis(dibenzylideneacetone)palladium(0) (Pd(dba)₂), palladium(2) acetate, and palladium(2) chloride, and the palladium catalyst may be tris(dibenzylideneacetone)dipalladium(0) in one preferred embodiment. The equivalent of the palladium catalyst in bringing the compound represented by the above formula (IV) or its protected product into contact with the compound represented by the above formula (V) may be, for example, 0.0005 to 0.2, 0.001 to 0.1, or 0.003 to 0.05 equivalents per equivalent of the compound represented by the above formula (IV) or its protected product.

In one embodiment, bringing the compound represented by the above formula (IV) or its protected product into contact with the compound represented by the above formula (V) is performed in the presence of a ligand in addition to the palladium catalyst. The ligand is not particularly limited as long as the compound represented by the above formula (II) or its protected product can be obtained by bringing the compound represented by the above formula (IV) or its protected product into contact with the compound represented by the above formula (V), examples of the ligand include 2-dicyclohexylphosphino-2',4',6'-triisopropylbiphenyl (XPhos), 1,1'-bis(diphenylphosphino)ferrocene (DPPF), 2,2'-bis(diphenylphosphino)-1,1'-binaphthyl (BINAP), 4,5-bis(diphenylphosphino)-9,9-dimethylxanthene (Xantphos), and 2-dicyclohexylphosphino-2',6'-diisopropoxybiphenyl (RuPhos), and the ligand may be, for example, 2-dicyclohexylphosphino-2',4',6'-triisopropylbiphenyl (XPhos) in one preferred embodiment. The equivalent of the ligand in bringing the compound represented by the above formula (IV) or its protected product into contact with the compound represented by the above formula (V) may be, for example, 0.005 to 0.08, 0.008 to 0.05, or 0.01 to 0.04 equivalents per equivalent of the compound represented by the above formula (IV) or its protected product.

In one embodiment, bringing the compound represented by the above formula (IV) or its protected product into contact with the compound represented by the above formula (V) is performed in the presence of a base in addition to the palladium catalyst and the ligand. The base is not particularly limited as long as the compound represented by the above formula (II) or its protected product can be obtained by bringing the compound represented by the above formula (IV) or its protected product into contact with the compound represented by the above formula (V), and the base may be potassium carbonate, cesium carbonate, potassium hydroxide, sodium tertiary butoxide, or sodium methoxide, for example. The equivalent of the base in bringing the compound represented by the above formula (IV) or its protected product into contact with the compound represented by the above formula (V) may be, for example, 1.5 to 20, 2.0 to 10, or 3.0 to 7.0 equivalents per equivalent of the compound represented by the above formula (IV) or its protected product.

In one embodiment, the second/third step includes, in the following order,
converting the compound represented by the above formula (IV) into a compound represented by the following formula (IX): 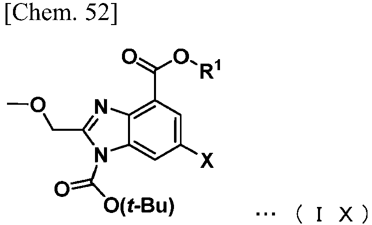
(wherein R¹ is the same as R¹ in the above formula (II), and X is the same as X in the above formula (IV)),
bringing the compound represented by the above formula (IX) into contact with the compound represented by the above formula (V) to obtain a compound represented by the following formula (X): 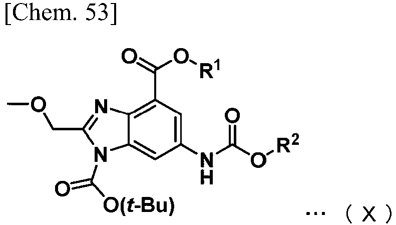
(wherein R¹ is the same as R¹ in the above formula (II), and R² is the same as R² in the above formula (V)), and
hydrolyzing the compound represented by the above formula (X) in the presence of an acid to obtain the compound represented by the above formula (II).

In this case, converting the compound represented by the above formula (IV) into the compound represented by the above formula (IX) can be performed by a method commonly used by those skilled in the art, and may be performed by bringing, for example, about 1.1 to 3 equivalents of di-tert-butyl dicarbonate ((Boc)₂O) into contact with, for example, 1 equivalent of the compound represented by the above formula (IV) and dissolved in an organic solvent such as toluene, at a temperature condition of room temperature to about 60°C. In this case, about 1.2 to 5 equivalents of a base (e.g., potassium carbonate) may be further present in the system.

In one embodiment, converting the compound represented by the above formula (IV) into the compound represented by the above formula (IX) and bringing the compound represented by the above formula (IX) into contact with the compound represented by the above formula (V) to obtain the compound represented by the above formula (X) in the second/third step may be performed in one reaction vessel (one pot). A specific example may be the case where, after the compound represented by the above formula (IV) is brought into contact with dit-butyl dicarbonate in an organic solvent such as toluene in which a base may be dissolved, the compound represented by the above formula (V), a palladium catalyst, a ligand, and a base are added to the reaction vessel, and the reaction solution is heated to obtain the compound represented by the above formula (X).

Hydrolyzing the compound represented by the above formula (X) in the presence of an acid to obtain the compound represented by the above formula (II) can be performed by a method commonly used by those skilled in the art, and can be performed, for example, by bringing an excess amount of an acid such as concentrated hydrochloric acid or trifluoroacetic acid into contact with the above formula (X) in isopropanol (2-propanol), methanol, or acetonitrile. In this case, the reaction solution may be heated to about 50 to 60°C if necessary.

In one embodiment, hydrolyzing the compound represented by the above formula (X) in the presence of an acid to obtain the compound represented by the above formula (II) may be performed without purification after bringing the compound represented by the above formula (IX) into contact with the compound represented by the above formula (V) to obtain the compound represented by the above formula (X). In this case, this can be performed by filtering the reaction solution obtained after bringing the compound represented by the above formula (IX) into contact with the compound represented by the above formula (V) to obtain the compound represented by the above formula (X), to remove the base such as potassium carbonate, etc., as insoluble material, reducing or drying the solvent by removing the solvent under reduced pressure if necessary, then adding the solvent used for hydrolysis in the presence of an acid, and the acid, and performing incubation.

### <Fourth step>

The fourth step is a step of converting a compound represented by the following formula (II): 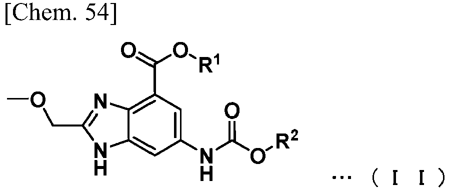 (wherein R¹ represents a protecting group for a carboxyl group or H, and R² represents a group that combines with -O-C(=O)- to form a protecting group for an aniline amino group) into a compound represented by the following formula (VIII):

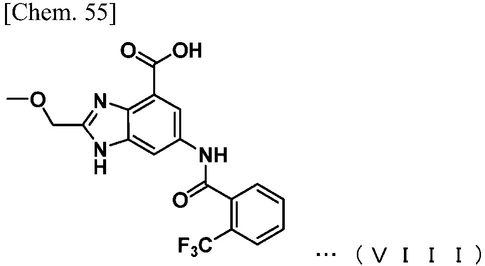

In one embodiment, the step of converting the compound represented by the above formula (II) into the compound represented by the above formula (VIII) includes converting the compound represented by the above formula (II) into a compound represented by the following formula (III): 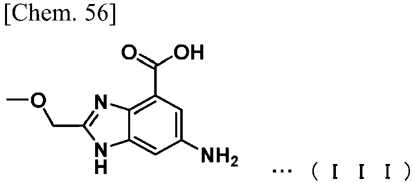 In one preferred embodiment, the step of converting the compound represented by the above formula (II) into the compound represented by the above formula (VIII) includes hydrolyzing the compound represented by the above formula (II) in the presence of a base to obtain the compound represented by the above formula (III).

Converting the compound represented by the above formula (II) into the compound represented by the above formula (III) can be performed by a method commonly used by those skilled in the art. According to the method including such a step, it is possible to avoid isolation of a compound (compound C) whose mutagenicity is a concern and which is represented by the following formula (XI): 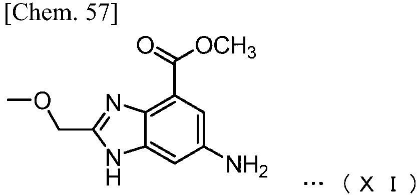 as a powder, so that the risk of mutagenicity due to scattering or the like of the compound C isolated as a powder can be avoided, and the cost in terms of time and money for worker protection and environmental countermeasures (containment) can be reduced. In one embodiment, the step of converting the compound represented by the above formula (II) into the compound represented by the above formula (VIII) includes converting the compound represented by the above formula (II) into the compound represented by the above formula (III), and the compound represented by the formula (III) is obtained as a solution thereof. In one embodiment, the step of converting the compound represented by the above formula (II) into the compound represented by the above formula (VIII) includes hydrolyzing the compound represented by the above formula (II) in the presence of a base to obtain the compound represented by the above formula (III) as a solution thereof. In one embodiment, the step of converting the compound represented by the above formula (II) into the compound represented by the above formula (VIII) does not include isolating the compound represented by the above formula (III) as a powder. In one embodiment, the step of converting the compound represented by the above formula (II) into the compound represented by the above formula (VIII) does not include handling the compound represented by the above formula (III) in a state other than a solution thereof.

Hydrolyzing the compound represented by the above formula (II) by a base can be performed under conditions commonly employed by those skilled in the art, and an example thereof is to add the compound represented by the above formula (II) or its salt (e.g., hydrochloride) to a sodium hydroxide solution having a concentration of about 5 to 30 mass% or 10 to 25 mass%, and stir the mixture at a temperature condition of about 40 to 70°C or 50 to 60°C. The reaction time is not particularly limited, may be, for example, 10 minutes to 72 hours, 1 to 24 hours, or 2 to 12 hours, and may be 6 hours as an example.

In one embodiment of the present disclosure, the step of converting the compound represented by the above formula (II) into the compound represented by the above formula (VIII) includes converting the compound represented by the above formula (III) into the compound represented by the above formula (VIII) after converting the compound represented by the above formula (II) into the compound represented by the above formula (III). Converting the compound represented by the above formula (III) into the compound represented by the above formula (VIII) can be performed by a method commonly used by those skilled in the art, and may be performed, for example, by a method of bringing an acid halide such as an acid chloride or acid bromide of 2-(trifluoromethyl)benzoic acid into contact with the compound represented by the above formula (VIII) to amidate the aniline amino group in the compound represented by the above formula (VIII), or may be performed by a method of bringing 2-(trifluoromethyl)benzoic acid into contact with the compound represented by the above formula (VIII) in the presence of a condensing agent such as HATU (1-[bis(dimethylamino)methylene]-1H-1,2,3-triazolo[4,5-b]pyridinium 3-oxide hexafluorophosphate) to amidate the aniline amino group in the compound represented by the above formula (VIII). Among them, from the viewpoint of avoiding a reduction in yield due to byproducts being produced due to the involvement of the carboxyl group of the compound represented by the above formula (VIII) in the amide formation, it is preferable that converting the compound represented by the above formula (III) into the compound represented by the above formula (VIII) is performed by the method of bringing an acid halide such as an acid chloride or acid bromide of 2-(trifluoromethyl)benzoic acid into contact with the compound represented by the above formula (VIII) to amidate the aniline amino group in the compound represented by the above formula (VIII), and this may be performed, for example, by bringing the compound represented by the above formula (VIII) into contact with 2-(trifluoromethyl)benzoyl chloride.

In the above case, a solvent normally selected by those skilled in the art may be used as a solvent in bringing an acid halide of 2-(trifluoromethyl)benzoic acid into contact with the compound represented by the above formula (III), and the solvent may be, for example, acetonitrile or a mixed solvent of acetonitrile and water. The temperature in the reaction is not particularly limited as long as the temperature is a temperature at which the compound represented by the above formula (VIII) is formed, and may be, for example, -10°C to 20°C, - 10°C to 10°C, or -5°C to 5°C, and the reaction time is also not particularly limited as long as the reaction time is a time for which the compound represented by the above formula (VIII) is formed, and may be, for example, 1 minute to 3 hours, 5 minutes to 1 hour, or 10 minutes to 30 minutes. In addition, from the viewpoint of increasing the yield by decomposing compounds obtained by reaction of multiple molecules of the acid halide of 2-(trifluoromethyl)benzoic acid which is a byproduct (acylated products obtained by reaction between NH of the imidazole ring or OH of the carboxylic acid bonded to benzimidazole and the halide of 2-(trifluoromethyl)benzoic acid), the reaction solution may be further incubated, for example, at a temperature of 50 to 80°C or 60 to 70°C, for example, for 5 minutes to 12 hours, 15 minutes to 6 hours, or 30 minutes to 4 hours after the reaction under the above conditions. In addition, the equivalent relationship between the acid halide of 2-(trifluoromethyl)benzoic acid and the compound represented by the above formula (III) is not particularly limited as long as the compound represented by the above formula (VIII) is obtained at a high yield (e.g., a yield of 80% or more) relative to the compound represented by the above formula (III). For example, the equivalent of the acid halide of 2-(trifluoromethyl)benzoic acid may be 0.90 to 1.50, 0.95 to 1.30, 0.97 to 1.10, or 0.99 to 1.01 equivalents per equivalent of the compound represented by the above formula (III). In addition, in the reaction system, a compound having a carboxyl group, such as acetic acid, may be further present in about 1 equivalent (e.g., 0.7 to 1.5 or 0.9 to 1.1 equivalents) relative to the compound represented by the above formula (III).

In one embodiment, converting the compound represented by the above formula (II) into the compound represented by the above formula (III) and converting the compound represented by the above formula (III) into the compound represented by the above formula (VIII) may be performed in one reaction vessel (one pot). In this case, for example, after the compound represented by the above formula (II) is converted into the compound represented by the above formula (III), acetonitrile, water, acetic acid, and an acid halide of 2-(trifluoromethyl)benzoic acid may be further added to the reaction solution.

### <Fifth step>

A fifth step is a step of converting a compound represented by the following formula (VIII): 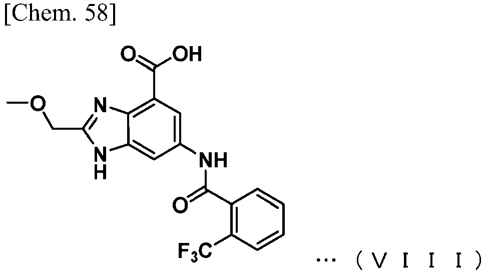 into an N,N-dimethylacetamide solvate of a compound (compound A) represented by the following formula (I): 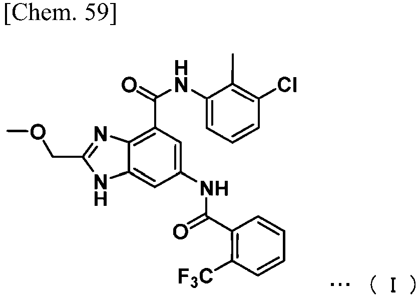 In one embodiment, the fifth step includes converting the compound represented by the above formula (VIII) into the compound (compound A) represented by the above formula (I), and bringing the compound (compound A) represented by the above formula (I) into contact with N,N-dimethylacetamide to obtain the N,N-dimethylacetamide solvate of the compound (compound A) represented by the above formula (I).

Converting the compound represented by the above formula (VIII) into the compound (compound A) represented by the above formula (I) can be performed by a method commonly used by those skilled in the art, and can be performed, for example, by bringing a compound obtained by bringing the compound represented by the above formula (VIII) into contact with 1,1'-carbonyldiimidazole (CDI) or the like and represented by the following formula (XII): 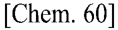 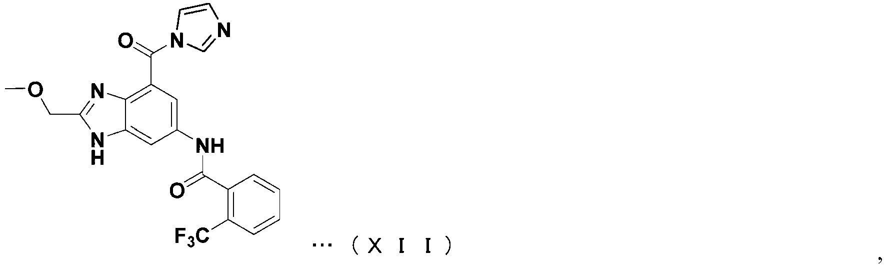 or an acid halide derivative such as a compound obtained by bringing the compound represented by the above formula (VIII) into contact with thionyl chloride or the like and represented by the following formula (XIII): 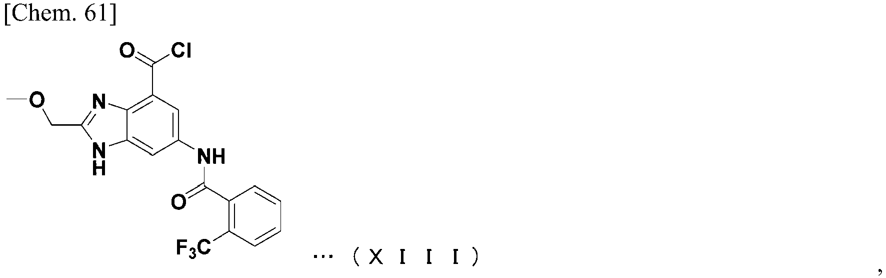 into contact with 3-chloro-2-methylaniline. For example, this can be performed by bringing the compound represented by the above formula (VIII) into contact with 3-chloro-2-methylaniline in the presence of a condensing agent such as HATU or DMTMM (4-(4,6-dimethoxy-1,3,5-triazin-2-yl)-4-methylmorpholinium chloride), and in one preferred embodiment, this can be performed by bringing the compound obtained by bringing the compound represented by the above formula (VIII) into contact with CDI and represented by the above formula (XII) into contact with 3-chloro-2-methylaniline.

The solvent in bringing the compound represented by the above formula (VIII) into contact with CDI may be a solvent that is not reactive with the compound represented by the above formula (VIII) and CDI, and, for example, N,N-dimethylformamide, N,N-dimethylacetamide, etc., are suitable for use as the solvent. In addition, the temperature and the time for bringing the compound represented by the above formula (VIII) into contact with CDI are not particularly limited as long as the compound represented by the above formula (XII) can be obtained, and the reaction may be carried out at a temperature condition of 5 to 50°C, 10 to 40°C or 20 to 30°C for 10 minutes to 24 hours, 20 minutes to 6 hours, or 30 minutes to 2 hours, for example. The equivalent relationship in bringing the compound represented by the above formula (VIII) into contact with CDI is not particularly limited as long as the compound represented by the above formula (XII) can be obtained at a high yield (e.g., a yield of 90% or more) from the compound represented by the above formula (VIII), and the equivalent of CDI may be, for example, 1.00 to 2.00, 1.05 to 1.40, or 1.10 to 1.20 equivalents per equivalent of the compound represented by the above formula (VIII).

The solvent in bringing the compound represented by the above formula (XII) into contact with 3-chloro-2-methylaniline may be a solvent that is not reactive with the compound represented by the above formula (XII) and 3-chloro-2-methylaniline, and, for example, N,N-dimethylformamide, N,N-dimethylacetamide, etc., are suitable for use as the solvent. In addition, the temperature and the time for bringing the compound represented by the above formula (XII) into contact with 3-chloro-2-methylaniline are not particularly limited as long as the compound (compound A) represented by the above formula (I) can be obtained, and reaction may be carried out at a temperature of 40 to 70°C or 50 to 60°C for 10 minutes to 168 hours, 1 to 72 hours, or 6 to 30 hours, for example. The equivalent relationship in bringing the compound represented by the above formula (XII) into contact with 3-chloro-2-methylaniline is not particularly limited as long as the compound (compound A) represented by the above formula (I) can be obtained at a high yield (e.g., a yield of 90% or more) relative to the compound represented by the above formula (XII), and the equivalent of 3-chloro-2-methylaniline may be, for example, 1.1 to 10, 1.5 to 5.0, or 2.0 to 3.0 per equivalent of the compound represented by the above formula (XII). In addition, bringing the compound represented by the above formula (XII) into contact with 3-chloro-2-methylaniline may be carried out in the presence of a compound having a carboxyl group such as such as benzoic acid.

Bringing the compound represented by the above formula (VIII) into contact with CDI and bringing the compound represented by the above formula (XII) into contact with 3-chloro-2-methylaniline may be performed in one reaction vessel (one pot). In this case, for example, this may be performed by bringing the compound represented by the above formula (VIII) into contact with CDI in N,N-dimethylformamide, and then adding 3-chloro-2-methylaniline and benzoic acid into the reaction solution.

Bringing the compound (compound A) represented by the above formula (I) into contact with N,N-dimethylacetamide to obtain the N,N-dimethylacetamide solvate of the compound (compound A) represented by the above formula (I) can be performed by a method commonly used by those skilled in the art, and can be performed, for example, by adding DMA or a mixed solution containing DMA (e.g., a mixed solution of DMA and water) to a solvent in which the compound represented by the above formula (I) in any form (e.g., the free form of the compound represented by the above formula (I)) is dissolved, and performing incubation. In one embodiment, this may be performed by adding a mixed solution of DMA and water to the solution after bringing the compound represented by the above formula (XII) into contact with 3-chloro-2-methylaniline. In this case, the temperature condition may be, for example, 40 to 70°C or 50 to 60°C, and the incubation time may be, for example, 10 minutes to 24 hours.

By the fourth step and the fifth step described above, the N,N-dimethylacetamide solvate of the compound (compound A) represented by the above formula (I) is obtained with the compound represented by the above formula (II), as a starting material. That is, in one embodiment, it can be said that a step of converting the compound represented by the above formula (II) into the N,N-dimethylacetamide solvate of the compound (compound A) represented by the above formula (I), is performed by a combination of the fourth step and the fifth step.

### <Sixth step>

A sixth step is a step of purifying a crude product of the N,N-dimethylacetamide solvate of the compound (compound A) represented by the above formula (I), by recrystallization. In one embodiment, the sixth step is a step of purifying the N,N-dimethylacetamide solvate of the compound (compound A) represented by the above formula (I) and obtained in the fifth step, by recrystallization. Recrystallization of the crude product of the N,N-dimethylacetamide solvate of the compound (compound A) represented by the above formula (I) may be performed in N,N-dimethylacetamide or a mixed solvent containing N,N-dimethylacetamide, and may be performed in a mixed solvent of N,N-dimethylacetamide and water in one embodiment. Also, in one preferred embodiment, recrystallization of the crude product of the N,N-dimethylacetamide solvate of the compound (compound A) represented by the above formula (I) may be performed in a mixed solvent of N,N-dimethylacetamide and water in which the content of N,N-dimethylacetamide is 50 vol% or more, 60 vol% or more, 70 vol% or more, 75 vol% or more, or 78 vol% or more and is 99 vol% or less, 95 vol% or less, 90 vol% or less, or 80 vol% or less, and may be performed, for example, in a mixed solvent of N,N-dimethylacetamide and water in which the content of N,N-dimethylacetamide is 75 vol% or more, or is 75 vol% or more and 99 vol% or less. If recrystallization is performed in such a solvent, the N,N-dimethylacetamide solvate of the compound (compound A) represented by the above formula (I) can be obtained at a high yield and a high purity. Also, if recrystallization is performed in such a solvent, a refined product, of the N,N-dimethylacetamide solvate of the compound (compound A) represented by the above formula (I), in which the content of a compound (hereinafter also referred to as "compound B") which is formed in the process of production, which may be involved in mutagenicity, and which is represented by the following formula (VII): 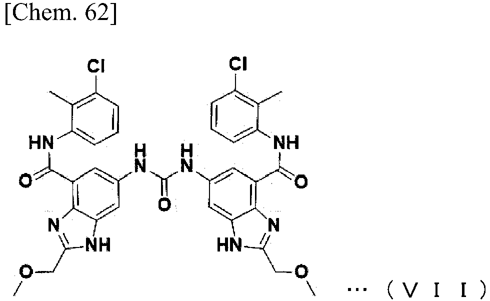 is reduced. In particular, if a mixed solvent of N,N-dimethylacetamide and water in which the content of N,N-dimethylacetamide is 70 vol% or more, 75 vol% or more, or 78 vol% or more is used, a refined product, of the N,N-dimethylacetamide solvate of the compound (compound A) represented by the above formula (I), in which the content of the compound B is further reduced, can be obtained. In these cases, the content of the compound B being reduced may be that the content of the compound B in the refined product (crystal) obtained by recrystallization is, for example, 1800×10⁻⁴ mass% or less, 1000×10⁻⁴ mass% or less, 500×10⁻⁴ mass% or less, 400×10⁻⁴ mass% or less, 300×10⁻⁴ mass% or less, 200×10⁻⁴ mass% or less, or 170×10⁻⁴ mass% or less, and, in particular, if a mixed solvent of N,N-dimethylacetamide and water in which the content of N,N-dimethylacetamide is 70 vol% or more, 75 vol% or more, or 78 vol% or more is used, a refined product (crystal) in which the content of the compound B is, for example, 1000×10⁻⁴ mass% or less, 500×10⁻⁴ mass% or less, 400×10⁻⁴ mass% or less, 300×10⁻⁴ mass% or less, 200×10⁻⁴ mass% or less, or 170×10⁻⁴ mass% or less, can be obtained.

### <Seventh step>

A seventh step is a step of converting the N,N-dimethylacetamide solvate of the compound (compound A) represented by the above formula (I) into a p-toluenesulfonate of the compound (compound A) represented by the above formula (I). In one embodiment, the seventh step is a step of converting the refined product (crystal) of the N,N-dimethylacetamide solvate of the compound (compound A) represented by the above formula (I) obtained in the sixth step into the p-toluenesulfonate of the compound (compound A) represented by the above formula (I). The seventh step is normally performed by bringing the N,N-dimethylacetamide solvate of the compound (compound A) represented by the above formula (I) into contact with p-toluenesulfonic acid (tosic acid, 4-methylbenzenesulfonate). The conditions for bringing the N,N-dimethylacetamide solvate of the compound (compound A) represented by the above formula (I) into contact with p-toluenesulfonic acid are not particularly limited as long as the N,N-dimethylacetamide solvate of the compound (compound A) represented by the above formula (I) is converted into the p-toluenesulfonate of the compound (compound A) represented by the above formula (I), and the solvent may be, for example, N,N-dimethylformamide, acetonitrile, or a mixed solvent thereof, and may be a mixed solvent of N,N-dimethylformamide and acetonitrile (e.g., a mixed solvent with a mass ratio of about 1:6) as a specific example. The temperature for this contact may be, for example, 30 to 70°C or 45 to 60°C. The time for this contact may be, for example, 10 minutes to 24 hours, or 1 hour to 12 hours. In addition, the form of p-toluenesulfonic acid to be added is not particularly limited, and p-toluenesulfonic acid may be added to the solvent as a hydrate, for example. As for the equivalent relationship in the reaction system, the equivalent of p-toluenesulfonic acid or its hydrate may be, for example, 1.01 to 2.00, 1.03 to 1.50, or 1.05 to 1.20 equivalents per equivalent of the N,N-dimethylacetamide solvate of the compound (compound A) represented by the above formula (I).

### <Eighth step>

An eighth step is a step of purifying a crude product of the p-toluenesulfonate of the compound (compound A) represented by the above formula (I). In one embodiment, the eighth step is a step of purifying a crude product of the p-toluenesulfonate of the compound (compound A) represented by the above formula (I) obtained in the seventh step. The eighth step includes purifying the p-toluenesulfonate of the compound (compound A) represented by the above formula (I) by bringing the p-toluenesulfonate into contact with activated carbon, and purifying the p-toluenesulfonate of the compound (compound A) represented by the above formula (I) after being brought into contact with the activated carbon, by recrystallization, in this order.

Purifying the p-toluenesulfonate of the compound (compound A) represented by the above formula (I) by bringing the p-toluenesulfonate into contact with activated carbon is performed by adding activated carbon to a solution of the p-toluenesulfonate of the compound (compound A) represented by the above formula (I), incubating the solution, and then removing the activated carbon by filtration or the like. The solvent is not particularly limited as long as the p-toluenesulfonate of the compound (compound A) represented by the above formula (I) is dissolved in the solvent and the form of the salt is maintained, and, for example, N,N-dimethylformamide is suitable for use as the solvent. The contact may be performed, for example, at a temperature condition of about 30 to 60°C or 40 to 50°C for about 5 minutes to 24 hours or 10 minutes to 6 hours, and a specific example is 1 hour. The amount of the activated carbon to be brought into contact with the p-toluenesulfonate of the compound (compound A) represented by the above formula (I) may be, for example, 0.04 times or more, 0.05 times or more, 0.08 times or more, 0.09 times or more, 0.10 times or more, 0.14 times or more, or 0.15 times or more the mass of the p-toluenesulfonate of the compound, may be 1.00 times or less, 0.50 times or less, or 0.20 times or less the mass of the p-toluenesulfonate of the compound, and may be 0.08 times or more the mass of the p-toluenesulfonate of the compound in one preferred embodiment. If the amount of the activated carbon to be brought into contact with the p-toluenesulfonate of the compound (compound A) represented by the above formula (I) is the above lower limit or more, the compound B, which is an impurity that may be involved in mutagenicity, can be efficiently removed.

Purifying the p-toluenesulfonate of the compound (compound A) represented by the above formula (I) after being brought into contact with the activated carbon, by recrystallization, can be performed by a method commonly used by those skilled in the art. In one embodiment, this may be performed by adding, if necessary, a seed crystal of the p-toluenesulfonate of the compound (compound A) represented by the above formula (I) to a filtrate obtained by removing the activated carbon by filtration after purifying the p-toluenesulfonate of the compound (compound A) represented by the above formula (I) by bringing the p-toluenesulfonate into contact with activated carbon, and then heating and cooling the filtrate.

If the eighth step includes purifying the p-toluenesulfonate of the compound (compound A) represented by the above formula (I) by bringing the p-toluenesulfonate into contact with activated carbon, and purifying the p-toluenesulfonate of the compound (compound A) represented by the above formula (I) after being brought into contact with the activated carbon, by recrystallization in this order, the content of the compound B, which is an impurity that may be involved in mutagenicity, in the refined product (crystal) obtained by recrystallization can be significantly reduced compared to the case where purification is performed by recrystallization alone. More specifically, the content of the compound B in the refined product (crystal) obtained by recrystallization can be reduced to 100×10⁻⁴ mass% or less, 70×10⁻⁴ mass% or less, 50×10⁻⁴ mass% or less, 40×10⁻⁴ mass% or less, 30×10⁻⁴ mass% or less, 20×10⁻⁴ mass% or less, 15×10⁻⁴ mass% or less, 12×10⁻⁴ mass% or less, or 10×10⁻⁴ mass% or less.

### <Embodiments of production method according to first aspect>

One embodiment of the first aspect of the present disclosure can be a production method for the p-toluenesulfonate of the compound (compound A) represented by the above formula (I), including at least one of the steps described above. One specific embodiment of the first aspect of the present disclosure can be a production method for the p-toluenesulfonate of the compound (compound A) represented by the above formula (I), including the seventh step. One embodiment of the first aspect of the present disclosure can be a production method for the p-toluenesulfonate of the compound (compound A) represented by the above formula (I), including the fourth step and the seventh step in this order. One embodiment of the first aspect of the present disclosure can be a production method for the p-toluenesulfonate of the compound (compound A) represented by the above formula (I), including the second/third step, the fourth step, and the seventh step in this order. One embodiment of the first aspect of the present disclosure can be a production method for the p-toluenesulfonate of the compound (compound A) represented by the above formula (I), including the fourth step, the fifth step, and the seventh step in this order. One embodiment of the first aspect of the present disclosure can be a production method for the p-toluenesulfonate of the compound (compound A) represented by the above formula (I), including the second/third step, the fourth step, the fifth step, and the seventh step in this order. One embodiment of the first aspect of the present disclosure can be a production method for the p-toluenesulfonate of the compound (compound A) represented by the above formula (I), including the first step, the second/third step, the fourth step, the fifth step, and the seventh step in this order. One embodiment of the first aspect of the present disclosure can be a production method for the p-toluenesulfonate of the compound (compound A) represented by the above formula (I), further including the sixth step immediately before the seventh step in these embodiments. One embodiment of the first aspect of the present disclosure can be a production method for the p-toluenesulfonate of the compound (compound A) represented by the above formula (I), further including the eighth step immediately after the seventh step in these embodiments.

### <Second aspect: N,N-dimethylacetamide solvate of compound A>

One embodiment of a second aspect of the present disclosure is an N,N-dimethylacetamide solvate of the compound A. In addition, one embodiment of the second aspect of the present disclosure can be a composition containing the N,N-dimethylacetamide solvate of the compound A, wherein the content of the N,N-dimethylacetamide solvate of the compound (compound A) represented by the above formula (I) is 95 mass% or more of the entire composition and the content of the compound B is a predetermined upper limit or less. The composition according to the second aspect of the present disclosure means a composition composed of one or more components contained in any material used in the process of preparing the N,N-dimethylacetamide solvate of the compound A and a product by a one-step or multi-step reaction therebetween.

Examples of a method for producing the p-toluenesulfonate of the compound (compound A) represented by the above formula (I) from the compound represented by the above formula (VIII) include a method for producing the p-toluenesulfonate of the compound (compound A) represented by the above formula (I), via the N,N-dimethylacetamide solvate of the compound (compound A) represented by the above formula (I), as in the present application (method via the N,N-dimethylacetamide solvate of the compound A), and a method for producing the p-toluenesulfonate of the compound (compound A) represented by the above formula (I), without involving the N,N-dimethylacetamide solvate of the compound (compound A) represented by the above formula (I), as described in Example 239 of PATENT DOCUMENT 1.

As for the method for producing the p-toluenesulfonate of the compound (compound A) represented by the above formula (I) from the compound represented by the above formula (VIII), in the method described in Example 239 of PATENT DOCUMENT 1, a purification step by chromatography (e.g., purification step by a silica gel column) for purifying the compound (compound A) represented by the above formula (I) as shown in the present application is required. However, with the method via the N,N-dimethylacetamide solvate of the compound A, for example, by proceeding through the step of recrystallizing the N,N-dimethylacetamide solvate of the compound (compound A) represented by the above formula (I), the p-toluenesulfonate of the compound (compound A) represented by the above formula (I) can be obtained in high purity without performing purification by chromatography.

The step of obtaining the compound represented by the above formula (VIII) is not limited to the method described above, and, for example, the method described in PATENT DOCUMENT 1 can also be used. That is, as the step of obtaining the compound represented by the above formula (VIII), for example, the method described above can be used, or the method described in PATENT DOCUMENT 1 can also be used. Even in the case where the compound represented by the above formula (VIII) is obtained by any of the above methods, by obtaining the p-toluenesulfonate of the compound (compound A) represented by the above formula (I) via the N,N-dimethylacetamide solvate of the compound (compound A) represented by the above formula (I), the p-toluenesulfonate of the compound (compound A) represented by the above formula (I) can be obtained with a low impurity content without performing purification by chromatography.

Due to the above, by obtaining the p-toluenesulfonate of the compound (compound A) represented by the above formula (I) via the N,N-dimethylacetamide solvate of the compound (compound A) represented by the above formula (I), the time and cost for purification by chromatography can be reduced, so that the impurity contained in the compound (compound A) represented by the above formula (I) and produced by any method can be efficiently removed.

Furthermore, in the process of producing the p-toluenesulfonate of the compound (compound A) represented by the above formula (I), by proceeding through the N,N-dimethylacetamide solvate of the compound (compound A) represented by the above formula (I), the p-toluenesulfonate of the compound (compound A) represented by the above formula (I) can be obtained with a low impurity content.

The content of the compound B in the composition according to one embodiment of the second aspect of the present disclosure may be, for example, 1800×10⁻⁴ mass% or less, 1000×10⁻⁴ mass% or less, 500×10⁻⁴ mass% or less, 400×10⁻⁴ mass% or less, 300×10⁻⁴ mass% or less, 200×10⁻⁴ mass% or less, or 170×10⁻⁴ mass% or less of the entire composition in terms of free form, and may be 200×10⁻⁴ mass% or less in one preferred embodiment. In addition, the content of the N,N-dimethylacetamide solvate of the compound A in the composition according to one embodiment of the second aspect of the present disclosure may be 95 mass% or more, 96 mass% or more, 97 mass% or more, 98 mass% or more, or 99 mass% or more of the entire composition.

The salt or composition according to one embodiment of the second aspect of the present disclosure may be produced by a production method including the sixth step of the first aspect of the present disclosure, may be produced by a production method including the fifth step and the sixth step of the first aspect of the present disclosure in this order, may be produced by a production method including the fourth step, the fifth step, and the sixth step of the first aspect of the present disclosure in this order, may be produced by a production method including the second/third step, the fourth step, the fifth step, and the sixth step of the first aspect of the present disclosure in this order, and may be produced by a production method including the first step, the second/third step, the fourth step, the fifth step, and the sixth step of the first aspect of the present disclosure in this order. The salt or composition according to one embodiment of the second aspect of the present disclosure may be recrystallized using a mixed solvent of N,N-dimethylacetamide and water, and, in one preferred embodiment, may be recrystallized using a mixed solvent of N,N-dimethylacetamide and water in which the content of N,N-dimethylacetamide is 75 vol% or more.

### <Third aspect: Composition containing p-toluenesulfonate of compound A>

One embodiment of a third aspect of the present disclosure is a composition containing the p-toluenesulfonate of the compound A, wherein the content of the p-toluenesulfonate of the compound A is 95 mass% or more of the entire composition and the content of the compound B is a predetermined upper limit or less. One embodiment of the third aspect of the present disclosure is a composition containing the p-toluenesulfonate of the compound A and the compound B, wherein the content of the p-toluenesulfonate of the compound A is 95 mass% or more of the entire composition, and the content of the compound B is a predetermined upper limit or less. The composition according to the third aspect of the present disclosure means a composition composed of one or more components contained in any material used in the process of preparing the p-toluenesulfonate of the compound A and a product by a one-step or multi-step reaction therebetween.

The content of the compound B in the composition according one embodiment of the third aspect of the present disclosure may be, for example, 1000×10⁻⁴ mass%, 600×10⁻⁴ mass%, 300×10⁻⁴ mass%, 100×10⁻⁴ mass%, 50×10⁻⁴ mass%, or 20×10⁻⁴ mass% of the entire composition in terms of free form, and, as a specific example, the content of the compound B may be 100×10⁻⁴ mass% or less in terms of free form. In addition, the content of the p-toluenesulfonate of the compound A in the composition according to one embodiment of the third aspect of the present disclosure may be 95 mass% or more, 96 mass% or more, 97 mass% or more, 98 mass% or more, or 99 mass% or more of the entire composition.

The composition according to one embodiment of the third aspect of the present disclosure may be produced by a production method including the eighth step of the first aspect of the present disclosure, may be produced by a production method including the seventh step and the eighth step of the first aspect of the present disclosure in this order, may be produced by a production method including the sixth step, the seventh step, and the eighth step of the first aspect of the present disclosure in this order, may be produced by a production method including the fifth step, the sixth step, the seventh step, and the eighth step of the first aspect of the present disclosure in this order, may be produced by a production method including the fourth step, the fifth step, the sixth step, the seventh step, and the eighth step of the first aspect of the present disclosure in this order, may be produced by a production method including the second/third step, the fourth step, the fifth step, the sixth step, the seventh step, and the eighth step of the first aspect of the present disclosure in this order, and may be produced by a production method including the first step, the second/third step, the fourth step, the fifth step, the sixth step, the seventh step, and the eighth step of the first aspect of the present disclosure. The composition according to one embodiment of the third aspect of the present disclosure may be purified by recrystallization after being purified by being brought into contact with activated carbon, may be purified, for example, by recrystallization after being purified by being brought into contact with activated carbon whose mass is 0.04 times or more the mass of the p-toluenesulfonate of the compound (compound A) represented by the above formula (I), and may be purified by recrystallization after being purified by being brought into contact with activated carbon whose mass is 0.08 times or more the mass of the p-toluenesulfonate of the compound (compound A) represented by the above formula (I) in one preferred embodiment.

### EXAMPLES

Hereinafter, the present disclosure will be described more specifically by means of examples, but the present disclosure is not limited to the examples below.

In the examples below, the following abbreviations are used.
IPC = In-Process Control test
dba = Dibenzylideneacetone
XPhos = 2-Dicyclohexylphosphino-2',4',6'-triisopropylbiphenyl
IPA = 2-Propanol
CDI = 1,1'-Carbonyldiimidazole
DMF = N,N-Dimethylformamide
DMAc, DMA = N,N-Dimethylacetamide
pTsOH-H₂O = p-Toluenesulfonic acid monohydrate
MeCN = Acetonitrile
HPLC = High performance liquid chromatography

¹H-NMR spectra in the examples were measured by a JNM-ECS400 type nuclear magnetic resonance apparatus (manufactured by JEOL RESONANCE Inc.). The observed peaks are expressed as chemical shift values δ (ppm) (s = singlet, d = doublet, t = triplet, q = quartet, brs = broad singlet, m = multiplet, dd = double doublet, dt = double triplet).

X-ray powder diffraction spectra in the examples were measured by MiniFlex 600 (manufactured by Rigaku Corporation) (voltage: 40 kv, current: 15 mA, wavelength: CuKα, solar slit: 5.0 degrees, scanning range: 4 to 40 degrees: scanning speed/counting time: 20.0).

### <Example 1: Production of compound 9 (p-toluenesulfonate of compound A)>

A compound 9 was produced by a production method outlined below.

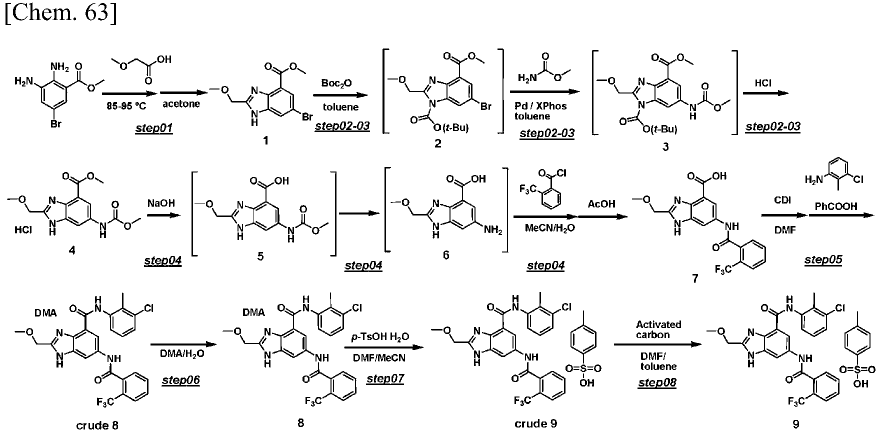

### [Step 1: Synthesis of methyl 6-bromo-2-(methoxymethyl)-1H-benzimidazole-4-carboxylate (compound 1)]

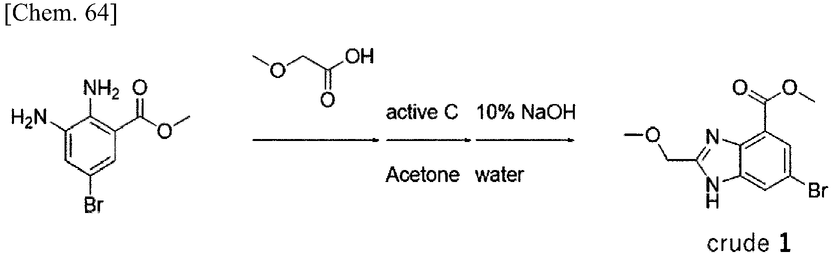

80.0 kg of methyl 2,3-diamino-5-bromobenzoate and methoxyacetic acid (294 kg) were added to a stirring tank purged with nitrogen. The temperature in the stirring tank was heated to 85°C or higher, and a reaction was carried out for 10 hours while maintaining the internal temperature at 80°C to 95°C. After confirming the completion of the reaction by HPLC based on the fact that the peak area of methyl 2,3-diamino-5-bromobenzoate, which was a raw material, reached 1% or less of the sum of the areas of all detected peaks, the reaction solution was cooled to 20 to 30°C. Then, acetone (506 kg) was added to the reaction solution, the reaction solution was stirred, then diatomaceous earth (8.0 kg), activated carbon (16.0 kg), and acetone (129 kg) were added to the reaction solution, and the reaction solution was further stirred for 1 hour or longer while maintaining the temperature of the reaction solution at 20 to 30°C. The insoluble material was filtered off, and the trapped insoluble material was washed with acetone (378 kg). Then, the collected filtrate was concentrated to 390 L by removing the solvent under reduced pressure while maintaining the temperature outside the vessel at 50°C or lower. Then, water (800 L) was added thereto while maintaining the temperature of the reaction solution at 20 to 50°C. Then, the reaction solution was cooled to 15°C or lower, and then about 1100 kg of a 10% sodium hydroxide solution was added such that the pH of the solution was 5.5 to 7.5, while maintaining the temperature of the reaction solution at 20°C or lower. The reaction solution was stirred for 30 minutes or longer while maintaining the temperature of the reaction solution at 20 to 30°C, then the slurry was filtered to collect the insoluble material, and the collected insoluble material was washed with water (602 kg), and then dried under reduced pressure while maintaining the temperature outside the vessel at 60°C or lower to obtain a crude product (crude) of methyl 6-bromo-2-(methoxymethyl)-1H-benzimidazole-4-carboxylate (compound 1), which was a target product, as 91.6 kg of a powder (yield: 94%).

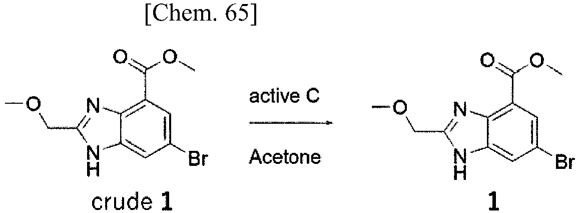

The obtained crude product of the compound 1 (91.5 kg) and acetone (1011 kg) were added to a stirring tank purged with nitrogen. The temperature in the stirring tank was raised to 35 to 45°C, and the compound 1 was dissolved in acetone. Activated carbon (9.2 kg) and acetone (73.2 kg) were added to the solution, and the reaction solution was then stirred for 1 hour or longer while maintaining the temperature of the reaction solution at 35 to 45°C. The insoluble material including the activated carbon was removed by filtration, and the trapped insoluble material was washed with acetone (434 kg). Then, the filtrate was concentrated to 640 L by removing the solvent while raising the temperature. The obtained solution was cooled to 20 to 30°C, and was stirred for 12 hours or longer while maintained at 20 to 30°C. Then, the solution was cooled to 10°C or lower over 1 hour or longer, and was stirred for 1 hour or longer while maintained at 0 to 10°C. The slurry was filtered to collect the insoluble material, and the collected insoluble material (cake) was washed with cooled acetone (73.1 kg). Then, the collected insoluble material was dried under reduced pressure while maintaining the temperature outside the vessel at 60°C or lower, to obtain methyl 6-bromo-2-(methoxymethyl)-1H-benzimidazole-4-carboxylate (compound 1) as 79.3 kg of a powder (yield: 87%).

The spectrum data of the obtained compound 1 was as follows.
ESI-MS (-): calculated value 297.0, measured value 297.0 (M-H)
1H NMR (Chloroform-d, δ in ppm) 10.47 (br, 1H), 8.04 (d, J = 1.3 Hz, 1H), 8.00 (d, J = 1.9 Hz, 1H), 4.76 (s, 2H), 4.00 (s, 3H), 3.51 (s, 3H)

### [Step 2/3: Synthesis of methyl 6-[(methoxycarbonyl)amino]-2-(methoxymethyl)-1H-benzimidazole-4-carboxylate hydrochloride (compound 4)]

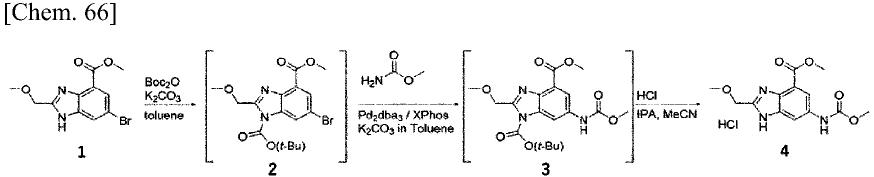

The compound 1 (79.2 kg) obtained in step 1, potassium carbonate (54.9 kg), and toluene (688 kg) were added to a stirring tank purged with nitrogen gas. Di-t-butyl dicarbonate (86.8 kg) was added to the mixture at a temperature of 15 to 30 °C, then the temperature of the reaction solution was raised to 40 to 50°C, and a reaction was carried out for 20 hours or longer. After confirming the completion of the reaction by HPLC based on the fact that the peak area of the compound 1, which was a raw material, reached 0.6% or less of the sum of the areas of all detected peaks, the reaction solution was cooled to 30°C to obtain a solution containing a compound 2 (compound 2 solution).

Methyl carbamate (39.8 kg), potassium carbonate (73.3 kg), and toluene (138 kg) were added to another stirring tank purged with nitrogen gas, and vacuum degassing was performed. The compound 2 solution and toluene (550 kg) were added to the mixture, and vacuum degassing was performed. 64.7%-Pd₂(dba)₃ (1.88 kg) and XPhos (2.54 kg) were added to the mixture, and vacuum degassing was performed. The reaction solution was heated to 88 to 97°C, and a reaction was carried out at 88 to 97°C for 4 hours. After confirming the completion of the reaction by HPLC based on the fact that the peak area of the compound 2, which was a raw material, reached 1.0% or less of the sum of the areas of all detected peaks, the reaction solution was cooled to 20 to 30°C. The insoluble material was removed by filtration, and the trapped insoluble material was washed with acetonitrile (437 kg). Then, 2-propanol (310 kg) was added to the collected filtrate containing a compound 3, and concentrated hydrochloric acid (81.6 kg) was further added thereto while maintaining the temperature of the solution at 30°C or lower. The temperature of the reaction solution was raised to 50 to 60°C, and a reaction was carried out for 1 hour or longer while maintaining the temperature at 50 to 60°C. After confirming the completion of the reaction by HPLC based on the fact that the peak area of the compound 3, which was a raw material, reached 0.1% or less of the sum of the areas of all detected peaks, the reaction solution was cooled to 25°C or lower and incubated at 15 to 25°C for 1 hour. The slurry was filtered to collect the insoluble material, and the collected insoluble material (cake) was washed with acetonitrile (683 kg). Then, the collected insoluble material was dried under reduced pressure while maintaining the temperature outside the vessel at 60°C or lower, to obtain methyl 6-[(methoxycarbonyl)amino]-2-(methoxymethyl)-1H-benzimidazole-4-carboxylate hydrochloride (compound 4) as 76.0 kg of a powder (yield: 87%).

The spectrum data of the obtained compound 4 was as follows.
ESI-MS(+): calculated value 294.1, measured value 294.0 (M+H)
1H NMR (DMSO-d6, δ in ppm) 10.19 (s, 1H), 8.24 (d, J = 1.9 Hz, 1H), 8.13 (d, J = 1.9 Hz, 1H), 4.94 (s, 2H), 3.96 (s, 3H), 3.68 (s, 3H), 3.44 (s, 3H)

### [Step 4: Synthesis of 2-(methoxymethyl)-6-(2-(trifluoromethyl)benzamido)-1H-benzimidazole-4-carboxylic acid (compound 7)]

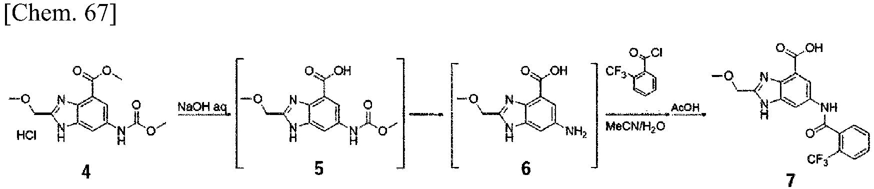

Water (76.2 kg) and a 30% sodium hydroxide solution (153 kg) were added to a stirring tank purged with nitrogen gas and were mixed. The compound 4 (75.6 kg) obtained in step 2/3 was added to the mixture, and degassing was performed with nitrogen gas. The reaction solution was heated to raise the temperature thereof to 50 to 60°C, and a reaction was carried out for 6 hours or longer while maintaining the temperature at 50 to 60°C. After confirming the completion of the reaction by HPLC based on the fact that the peak area of a compound 5, which was a reaction intermediate, reached 1.0% or less of the sum of the areas of all detected peaks, the reaction solution was cooled to 30°C, and water (756 L) and acetic acid (13.7 kg) were added to the reaction solution. Acetonitrile (356 kg) was added to the reaction solution, and the solution was cooled to 0°C or lower. 47.9 kg of 2-(trifluoromethyl)benzoyl chloride was added dropwise to the solution at -5 to 5°C, and a reaction was carried out for 15 minutes or longer while maintaining the temperature at -5 to 5°C. After confirming the completion of the reaction by HPLC based on the fact that the peak area of a compound 6, which was a reaction intermediate, reached 1.0% or less of the sum of the areas of all detected peaks, the reaction solution was heated to raise the temperature thereof to 60°C or higher, and a reaction was carried out for 2 hours or longer while maintaining the temperature at 60 to 70°C. After confirming the completion of the reaction by HPLC based on the fact that the sum of the peak areas of compounds obtained by reaction of two or three molecules of 2-(trifluoromethyl)benzoyl chloride, which was a byproduct of the previous reaction, with the compound 6 reached 0.1% or less of the sum of the areas of all detected peaks, acetonitrile (238 kg) was added at a temperature of 70°C or lower, and acetic acid (55.0 kg) was added over 30 minutes or longer while maintaining the temperature at 60 to 70°C. The reaction solution was stirred for 1 hour or longer while maintained at 60 to 70°C, then cooled to 30°C or lower, and further stirred for 1 hour or longer while maintained at 20 to 30°C. The slurry was filtered to collect the insoluble material, and the collected insoluble material (cake) was washed with a mixed solution of MeCN (142 kg) and water (182 kg), water (183 kg), and 2-propanol (144 kg) in this order. Then, the collected insoluble material was dried under reduced pressure while maintaining the temperature outside the vessel at 60°C or lower, to obtain 2-(methoxymethyl)-6-(2-(trifluoromethyl)benzamido)-1H-benzimidazole-4-carboxylic acid (compound 7) as 85.1 kg of a powder (yield: 94%).

The spectrum data of the obtained compound 7 was as follows.
ESI-MS(+): calculated value 394.1, measured value 394.0 (M+H)
1H NMR (Methanol-d4, δ in ppm) 8.28 (d, J = 2.6 Hz, 1H), 8.12 (d, J = 1.9 Hz, 1H), 7.64-7.81 (m, 4H), 4.72 (s, 2H), 3.46 (s, 3H)

### [Step 5: Synthesis of N-(3-chloro-2-methylphenyl)-2-(methoxymethyl)-6 -({[2-(trifluoromethyl)phenyl]carbonyl}amino)-1H-benzimidazole-4-carboxamide DMA solvate (compound 8)]

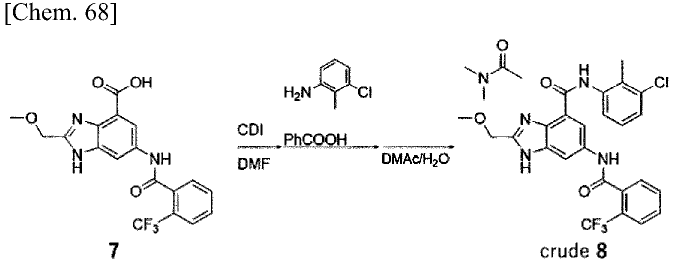

The compound 7 (82.8 kg) obtained in step 4 and DMF (235 kg) were added to a stirring tank purged with nitrogen gas. 1,1'-carbonyldiimidazole (38.8 kg) was added to the solution while stirring the solution at 30°C or lower, and a reaction was carried out for 1 hour or longer while maintaining the temperature of the reaction solution at 20 to 30°C. After confirming the completion of the reaction by HPLC based on the fact that the peak area of the compound 7, which was a starting material, reached 1.0% or less of the sum of the areas of all detected peaks, the reaction solution was cooled to 20 to 30°C, and 74.3 kg of 3-chloro-2-methylaniline and benzoic acid (1.28 kg) were added to the reaction solution. The reaction solution was heated to raise the temperature thereof, and a reaction was carried out at 50 to 60°C for 20 hours or longer. After confirming the completion of the reaction by HPLC based on the fact that the peak area of a reaction intermediate having a carbonyl imidazole structure reached 2.0% or less of the sum of the areas of all detected peaks, DMAc (465 kg) was added to the reaction solution at 50 to 60°C. Water (331 kg) was added dropwise to the reaction solution while maintaining the temperature of the reaction solution at 50 to 60°C, and then the reaction solution was stirred at 50 to 60°C for 1 hour or longer. The reaction solution was cooled to 25°C or lower, and was stirred for 2 hours or longer while maintaining the temperature of the reaction solution at 20 to 25°C. The slurry was filtered to collect the insoluble material, and the collected insoluble material (cake) was washed with a mixed solution of DMAc (183 kg) and water (195 kg), and water (293 kg) in this order. Then, the collected insoluble material was dried under reduced pressure while maintaining the temperature outside the vessel at 60°C or lower, to obtain a crude product of N-(3-chloro-2-methylphenyl)-2-(methoxymethyl)-6-({[2-(trifluoromethyl)phenyl]carbonyl}amino)-1H-benzimidazole-4-carboxamide DMA solvate (compound 8) as 115 kg of a powder (yield: 91%).

### [Step 6: Purification of N-(3-chloro-2-methylphenyl)-2-(methoxymethyl)-6-({[2-(trifluoromethyl)phenyl]carbonyl}amino)-1H-benzimidazole-4-carboxamide DMA solvate (compound 8)]

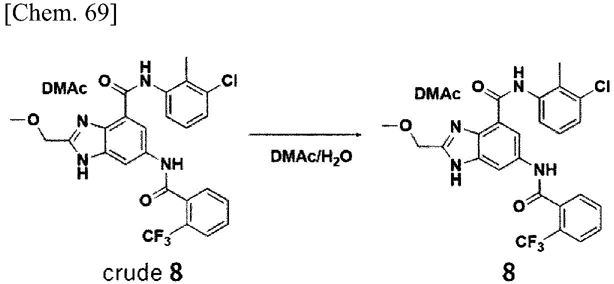

The crude product of the compound 8 (105 kg) obtained in step 5 and DMAc (322 kg) were added to a stirring tank. The mixed solution was heated to raise the temperature thereof to 70 to 80°C, and water (90.3 kg) was added to the solution at 70 to 80°C. The mixed solution was cooled to 25°C or lower over 90 minutes or longer, and was stirred at 20 to 30°C for 30 minutes or longer. The slurry was filtered to collect the insoluble material, and the collected insoluble material (cake) was washed with a mixed solution of DMAc (98.4 kg) and water (105 kg) and then water (211 kg). Then, the collected insoluble material was dried under reduced pressure while maintaining the temperature outside the vessel at 60°C or lower, to obtain N-(3-chloro-2-methylphenyl)-2-(methoxymethyl)-6-({[2-(trifluoromethyl)phenyl]carbonyl}amino)-1H-benzimidazole-4-carboxamide DMA solvate (compound 8) as 98.7 kg of a powder (yield: 94%).

The spectrum data of the obtained compound 8 was as follows.
ESI-MS(+): calculated value for the desolvated free form 517.1, measured value 517.0 (M+H)
¹HNMR (DMSO-d6, δ in ppm) 11.88 (s, 1H), 10.80 (s, 1H), 8.35 (d, J = 2.3 Hz, 1H), 8.24 (dd, J = 6.9 and 2.3 Hz, 1H), 8.13 (d, J = 1.8 Hz, 1H), 7.67-7.84 (m, 4H), 7.21-7.27 (m, 2H), 4.75 (s, 2H), 3.40 (s, 3H), 2.90 (s, 3H), 2.74 (s, 3H), 2.53 (s, 3H), 1.92 (s, 3H)

### [Step 7: Synthesis of N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate (compound 9)]

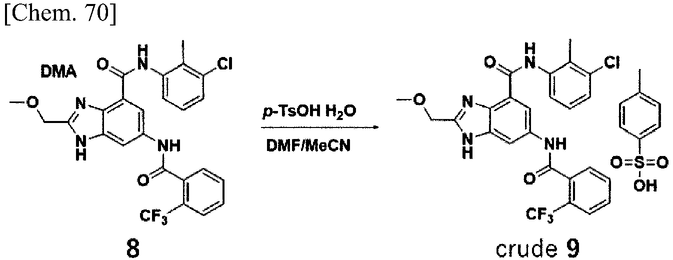

The compound 8 (98.0 kg) obtained in step 6 and DMF (231 kg) were added to a stirring tank. The solution was heated to raise the temperature thereof to 50 to 60°C, and the compound 8 was dissolved in DMF. Then, MeCN (576 kg) was added to the solution at 45 to 60°C. In a state of being maintained at 45 to 60°C, a solution of pTsOH-H2O (33.9 kg) dissolved in MeCN (307 kg) was added dropwise to this solution, and MeCN (461 kg) was further added to the solution while maintaining the temperature at 45 to 60°C. Then, the reaction solution was stirred for 5 hours or longer while maintained at 50 to 60°C. The slurry was filtered to collect the insoluble material, and the collected insoluble material (cake) was washed with a mixed solution of DMF (23.1 kg) and MeCN (134 kg) and then MeCN (230 kg). Then, the collected insoluble material was dried under reduced pressure while maintaining the temperature outside the vessel at 60°C or lower, to obtain a crude product of N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate (compound 9) as 96.5 kg of a powder (yield: 86%).

### [Step 8: Purification of N-(3-chloro-2-methylphenyl)-2-(methoxymethyl)-6-({[2-(trifluoromethyl)phenyl]carbonyl} amino)-1H-benzimidazole-4-carboxamide 4-methylbenzenesulfonate (compound 9)]

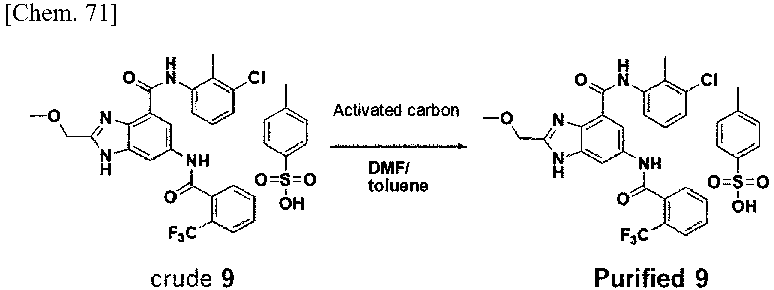

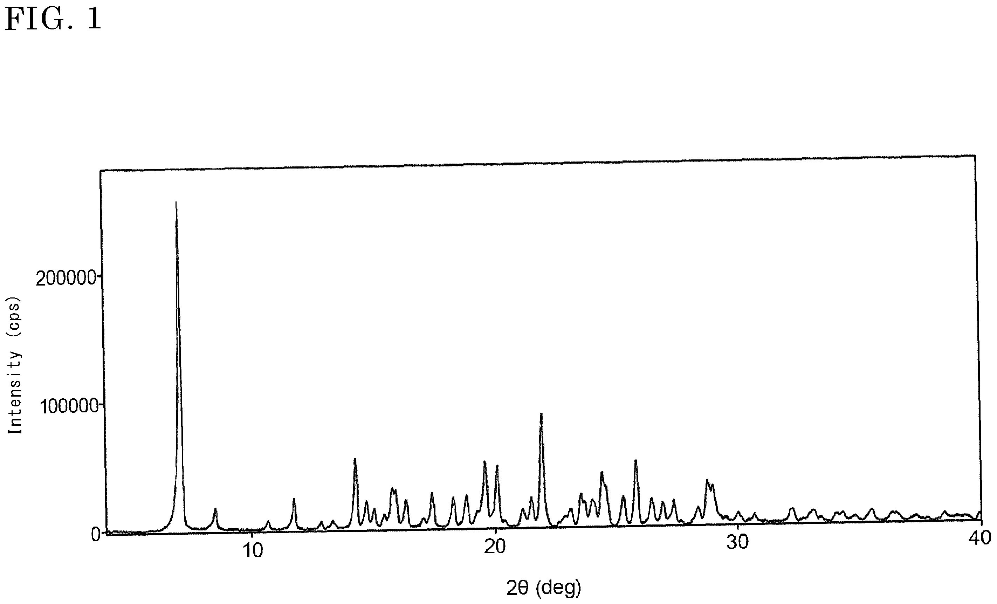

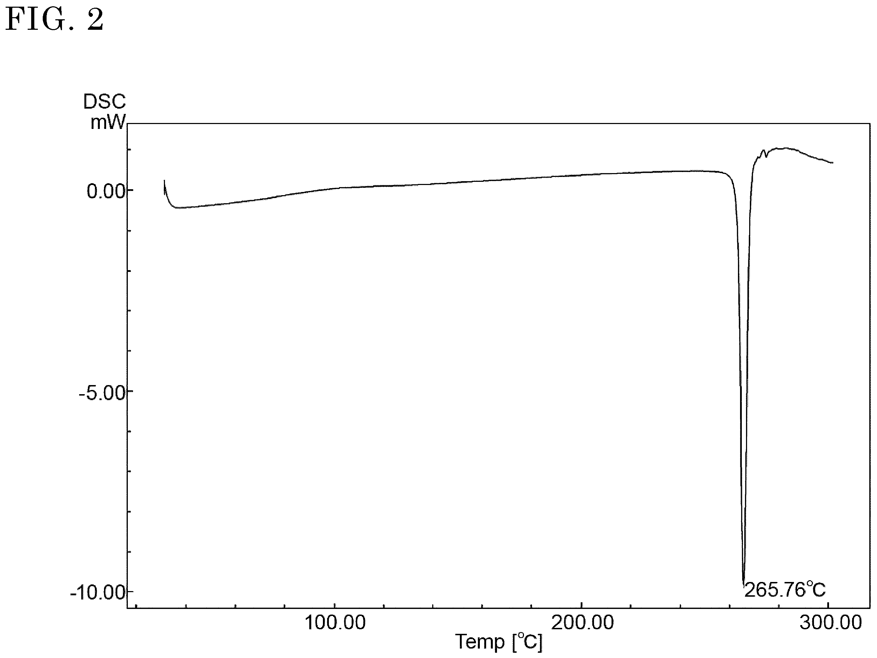

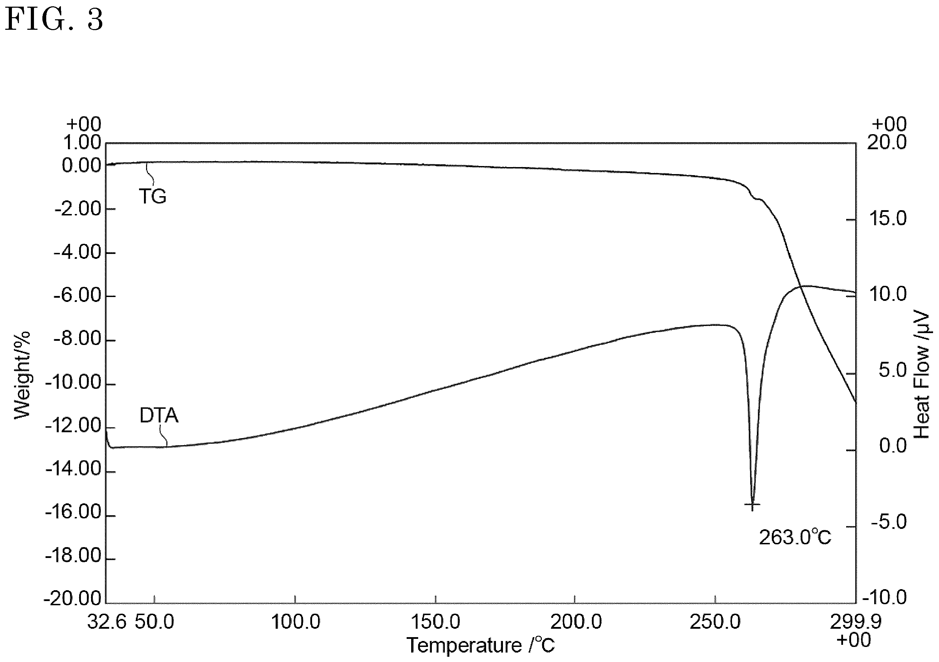

The crude product of the compound 9 (48.0 kg) obtained in step 7 and DMF (113 kg) were added to a stirring tank, and the temperature inside the stirring tank was raised to 35 to 40°C to dissolve the crude product. Activated carbon (4.8 kg) was added to the solution, and then the reaction solution was heated to raise the temperature thereof to 40 to 50°C, and was stirred for 1 hour while maintained at 40 to 50°C. The slurry was filtered to remove the insoluble material, and the trapped insoluble material was washed with a mixed solution of DMF (22.6 kg) and toluene (41.5 kg). Toluene (208 kg) was added to the obtained filtrate at a temperature of 20 to 30°C. A seed crystal of the compound 9 (48.0 g) was added to the filtrate, toluene (872 kg) was then added dropwise thereto at a temperature of 20 to 30°C, and the solution was stirred for 5 hours or longer while maintained at 20 to 30°C. A seed crystal of the compound 9 (240 g) was added to the solution, and the solution was then heated to raise the temperature thereof to 50 to 60°C, and was stirred at 50 to 60°C for 5 hours or longer. The solution was cooled to 20 to 30°C, and was then further stirred at 20 to 30°C for 12 hours or longer. The slurry was filtered to collect the insoluble material, and the collected insoluble material (cake) was washed with toluene (125 kg) and then MeCN (75.2 kg). Then, the collected insoluble material was dried under reduced pressure while maintaining the temperature outside the vessel at 60°C or lower, to obtain N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate (compound 9) as 44.9 kg of a powder (yield: 94%). The results of X-ray powder crystal analysis of the obtained powder are shown in FIG. 1. According to FIG. 1, peaks were observed at 2θ = 7.1°, 14.3°, 15.8°, and 18.3°. In addition, the results of thermophysical property measurement by DSC of the obtained powder are shown in FIG. 2, and the results of thermophysical property measurement by TG-DTA of the obtained powder are shown in FIG. 3. Furthermore, the obtained powder was analyzed by HPLC, and as a result, the purity of the compound 9 based on the peak area at 230 nm was 99.95%. Moreover, the amounts of toluene and N,N-dimethylformamide as solvents remaining in the obtained compound 9 were 460 ppm and 514 ppm, respectively, and no acetonitrile was detected.

### [Step 9: Pulverization of powder of N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate (compound 9)]

The powder of the compound 9 (44.7 kg) obtained in step 8 was finely pulverized using a pulverizing machine (jet mill) to obtain 43.9 kg of a fine powder of N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate (compound 9) (yield: 98%).

### [Obtaining of crystal of compound 9 without using seed crystal]

N-(3-chloro-2-methylphenyl)-2-(methoxymethyl)-6-({[2-(trifluoromethyl)phenyl]carbonyl}amino)-1H-benzimidazole-4-carboxamide can be obtained by the method described in Example 11 of PATENT DOCUMENT 1. 480 ml of THF was added to 80 g of this N-(3-chloro-2-methylphenyl)-2-(methoxymethyl)-6-({[2-(trifluoromethyl)phenyl]carbonyl}amino)-1H-benzimidazole-4-carboxamide, and the mixture was heated at 50°C for dissolution. 32.4 g of p-toluenesulfonic acid monohydrate was added thereto, the mixture was stirred at the same temperature for 1 hour or longer, and 960 ml of acetone was added thereto at 55°C while heating. The mixture was further stirred at 50°C for 2 hours or longer while heating, then cooled to room temperature, and stirred at the same temperature for 30 minutes. The slurry was filtered, and washing was performed with 50 ml of cold acetone. Drying was performed under reduced pressure while heating at an external temperature of 50°C or lower, to obtain 98.1 g of a white crystal at a yield of 92%.

A portion of a white crystal obtained in the same manner as the obtaining method described above can be used as a seed crystal of the compound 9.

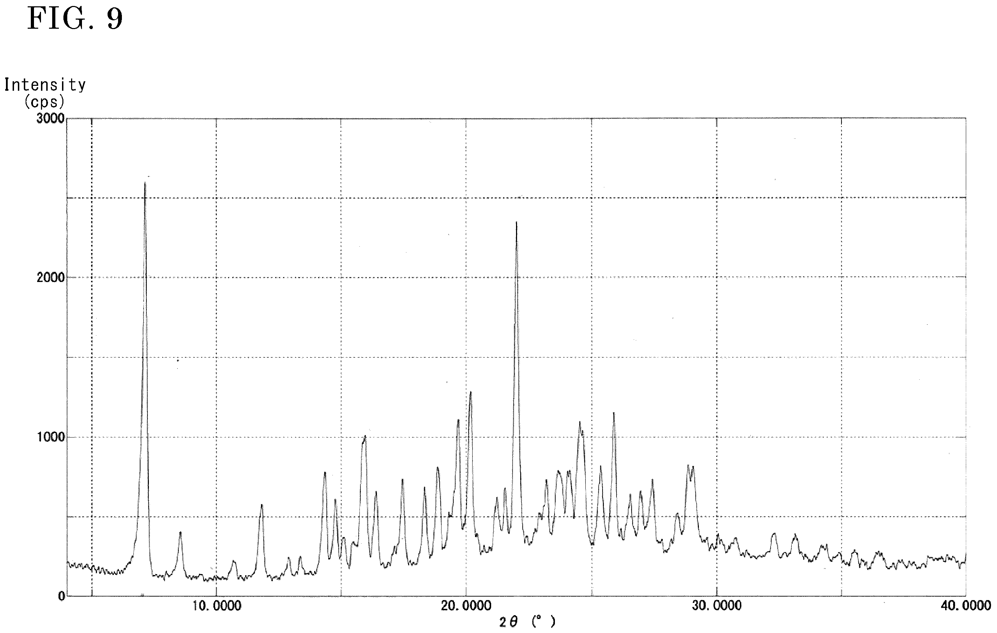

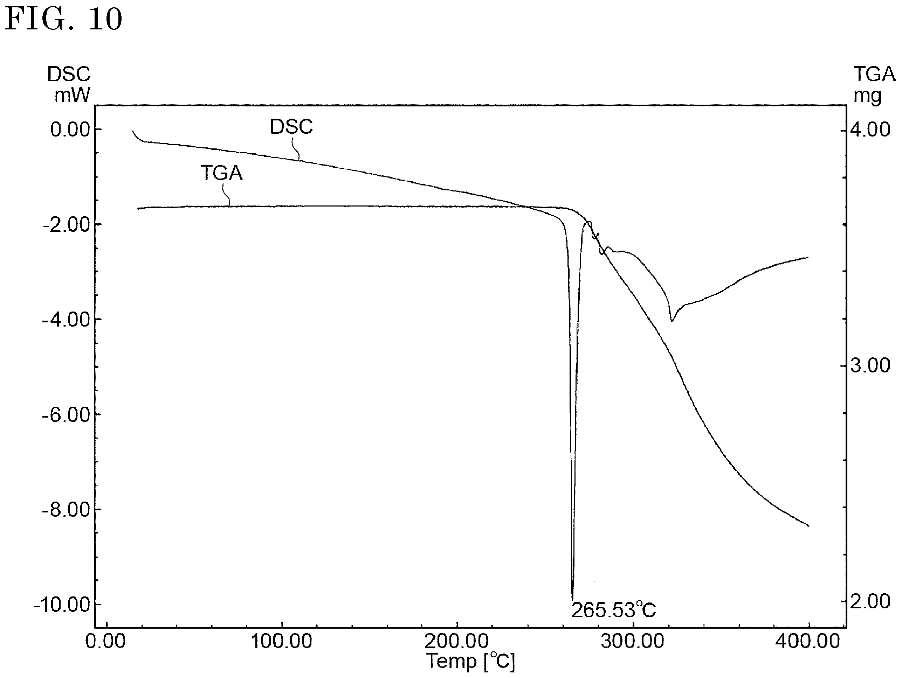

The results of X-ray powder diffraction of the obtained crystal of the compound 9 are shown in FIG. 9. The results of thermophysical property measurement by differential scanning calorimetry (DSC) and thermogravimetric analysis (TGA) of the obtained crystal of the compound 9 are shown in FIG. 10. An X-ray powder diffraction spectrum in Reference Example 1 was measured by RINT-Ultima III (manufactured by Rigaku Corporation) (target: Cu, voltage: 40 kv, current: 40 mA, scanning speed: 4 degrees/min). DSC in Reference Example 1 was measured by DSC-50 (manufactured by SHIMADZU CORPORATION) (cell: alumina (open), gas: nitrogen (20.0 mL/min), heating rate: 10.0°C/min, hold temperature: 400°C, hold time: 0 min).

### <Example 2: Purification efficiency and impurity removal efficiency in step 6>

For the powder of the compound 8 or its crude product, the content of the compound B represented by the following formula, which is an impurity whose content is required to be controlled in the order of ppm, was measured before purification in step 6 was performed, after purification in step 6 was performed using a solvent in which the volume ratio of DMA to H₂O was 79:21, and after purification in step 6 was performed using a solvent in which the volume ratio of DMA to H₂O was 69:31.

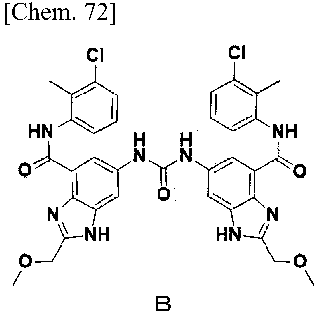

The results are shown in Table 1 below. According to Table 1, even when recrystallization was performed under any of the solvent conditions, the refined product of the compound 8 was obtained at a high yield, and the content of the compound B, which is an impurity, decreased. Furthermore, when the solvent conditions were compared, the content of the compound B was significantly decreased under the condition that the volume ratio of DMA to H₂O was 79:21. That is, the removal efficiency of the compound B was higher under the condition that the volume ratio of DMA to H₂O was 79:21. This indicates that, if N-(3-chloro-2-methylphenyl)-2-(methoxymethyl)-6-({[2-(trifluoromethyl)phenyl]carbonyl}amino)-1H-benzimidazole-4-carboxamide, which is a synthetic intermediate of the compound 9, is obtained as a DMA solvate (compound 8), the impurity that is required to be controlled in the order of ppm can be removed with high efficiency by the recrystallization method.

**[Table 1]**

| Purification in step 6 | DMA/H2O ratio (v/v) | Compound B (ppm) | Yield (%) |
|---|---|---|---|
| Before | - | 1855 | - |
| After | 3.27/0.86 (79:21) | 158 | 96.7 |
| After | 3.27/1.47 (69:31) | 1675 | 99.1 |

### <Example 3: Purification efficiency and impurity removal efficiency in step 8>

For the compound 8 obtained before purification in step 8, after purification was performed in step 8 only by recrystallization without contact with activated carbon, and after purification was performed by recrystallization after contact with activated carbon whose mass was 0.05 times, 0.10 times, or 0.15 times the mass of the crude product of the compound 8 as in step 8, the content of the compound B, which is an impurity, was measured.

The results are shown in Table 2 below. Unexpectedly, the compound B could not be effectively removed by recrystallization of the compound 8 alone. On the other hand, when contact with activated carbon was included before recrystallization, the compound B could be effectively removed, and, especially when the mass of activated carbon contacted was 0.10 times and 0.15 times the mass of the crude product of the compound 8, the compound B could be more efficiently removed.

**[Table 2]**

| Activated carbon treatment | Recrystallization | Compound B (ppm) |
|---|---|---|
| No | No | 112 |
| No | Yes | 108 |
| Yes, 5 wt% | Yes | 31.0 |
| Yes, 10 wt% | Yes | 13.8 |
| Yes, 15 wt% | Yes | 9.73 |

### <Example 4: Retest of step 5 to step 7>

Step 5 to step 7 were retested in the same manner as in Example 1. The compound prepared according to steps 1 to 4 of Example 1 was used as the compound 7, which was a starting material in step 5.

### [Step 5]

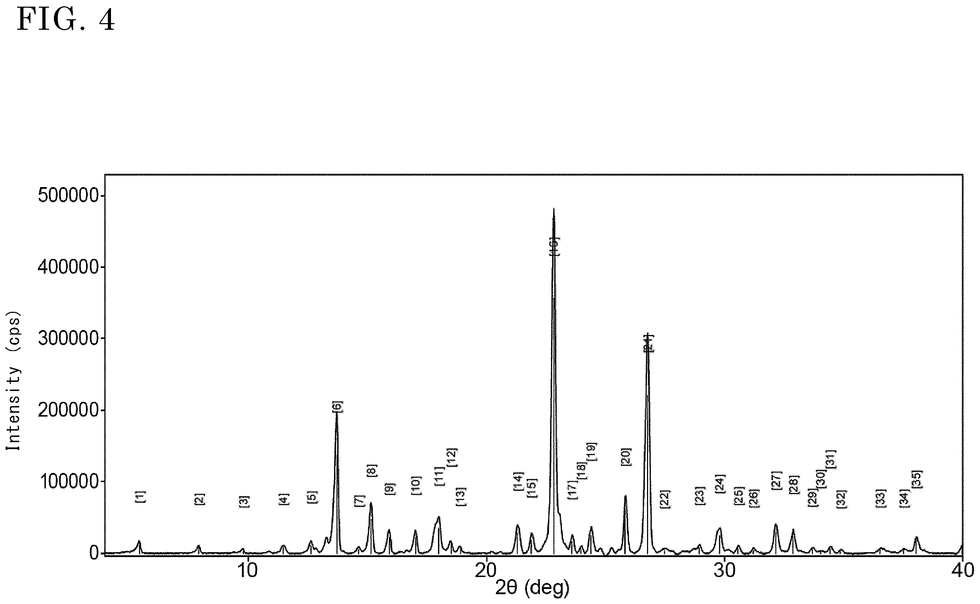

The compound 7 (58.8 kg) and DMF (167 kg) were added to a stirring tank purged with nitrogen gas. 1,1'-carbonyldiimidazole (26.3 kg) was added to the mixture while stirring the mixture at 30°C or lower, and a reaction was carried out at a temperature condition of 20°C to 30°C for 1 hour or longer. After analyzing the reaction solution by HPLC and confirming the completion of the reaction based on the fact that the peak area of the compound 7, which was a starting material, reached 1.0% or less of the sum of the areas of all detected peaks, 3-chloro-2-methylaniline (52.8 kg) and benzoic acid (0.91 kg) were added to the reaction solution in a state where the temperature thereof was at 20°C to 30°C. The temperature of the reaction solution was raised to 50 to 60°C, and a reaction was carried out for 20 hours or longer. After analyzing the reaction solution by HPLC and confirming the completion of the reaction based on the fact that the peak area of a reaction intermediate having a carbonyl imidazole structure reached 2.0% or less of the sum of the areas of all detected peaks, DMAc (331 kg) was added in a state where the reaction solution was at 50 to 60°C. Water (235 kg) was added dropwise to the reaction solution while maintaining the temperature of the reaction solution at 50 to 60°C, and then the reaction solution was stirred at a temperature condition of 50 to 60°C for 1 hour or longer. Then, the reaction solution was cooled to 25°C or lower, and was further stirred at a temperature of 20 to 25°C for 2 hours or longer. The slurry was filtered to collect the insoluble material, and the collected insoluble material (cake) was washed with a mixed solution of DMAc (133 kg) and water (142 kg), and water (212 kg) in this order. Then, the collected insoluble material was dried under reduced pressure while maintaining the temperature outside the vessel at 60°C or lower, to obtain the compound 8 as 79.8 kg of a powder (yield: 89%). The results of X-ray powder crystal analysis of the obtained powder are shown in FIG. 4.

### [Step 6]

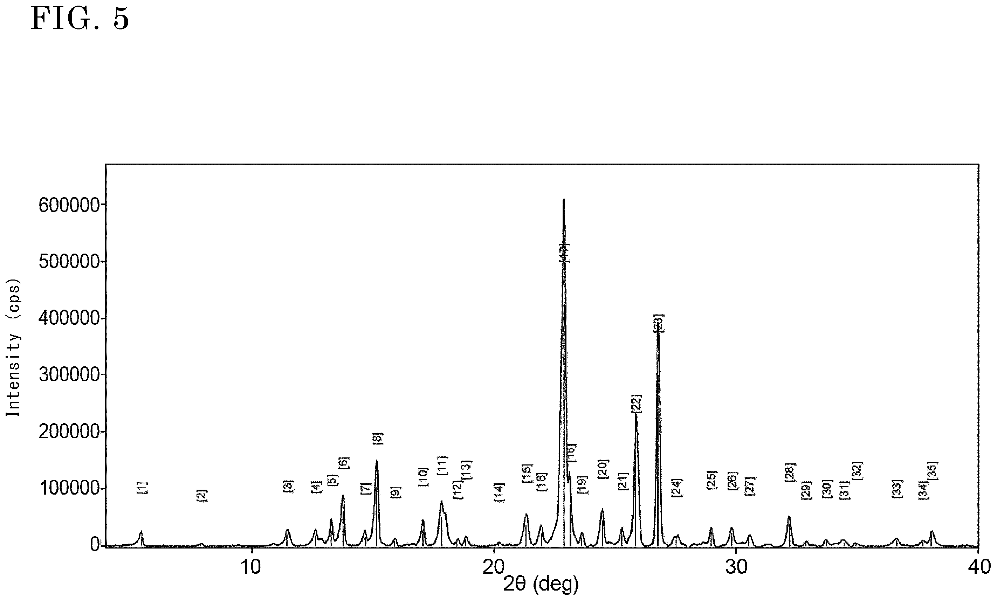

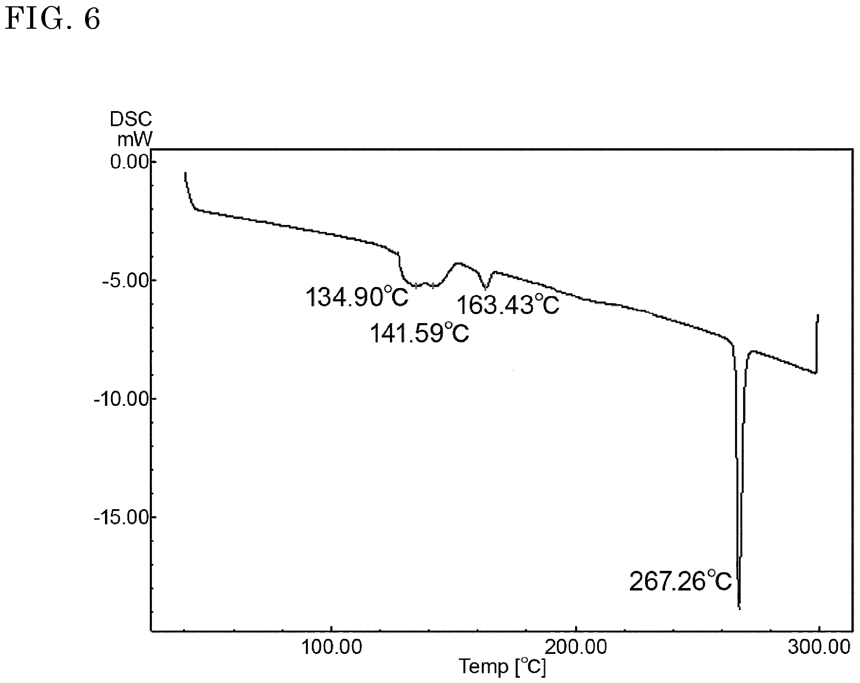

The crude product of the compound 8 (39.5 kg) obtained in step 5 and DMAc (122 kg) were added to a stirring tank. The temperature of the obtained solution was raised to 70 to 80°C, and water (34.0 kg) was added at 70 to 80°C. The solution was cooled to 25°C or lower over 90 minutes or longer, and was stirred at 20 to 30°C for 30 minutes or longer. The slurry was filtered to collect the insoluble material, and the collected insoluble material (cake) was washed with a mixed solution of DMAc (37.0 kg) and water (39.5 kg), and then water (79.0 kg). Then, the collected insoluble material was dried under reduced pressure while maintaining the temperature outside the vessel at 60°C or lower, to obtain a refined product of the compound 8 as 37.2 kg of a powder (yield: 94%). The results of X-ray powder crystal analysis of the obtained powder are shown in FIG. 5. The results of thermophysical property measurement by a differential scanning calorimeter (DSC) of the obtained powder are shown in FIG. 6. The DSC shown in FIG. 6 was measured by DSC-60A (manufactured by SHIMADZU CORPORATION) (cell: alumina (open), gas: nitrogen (20.0 mL/min), heating rate: 10.0°C/min, hold temperature: 300°C, hold time: 0 min).

### [Step 7]

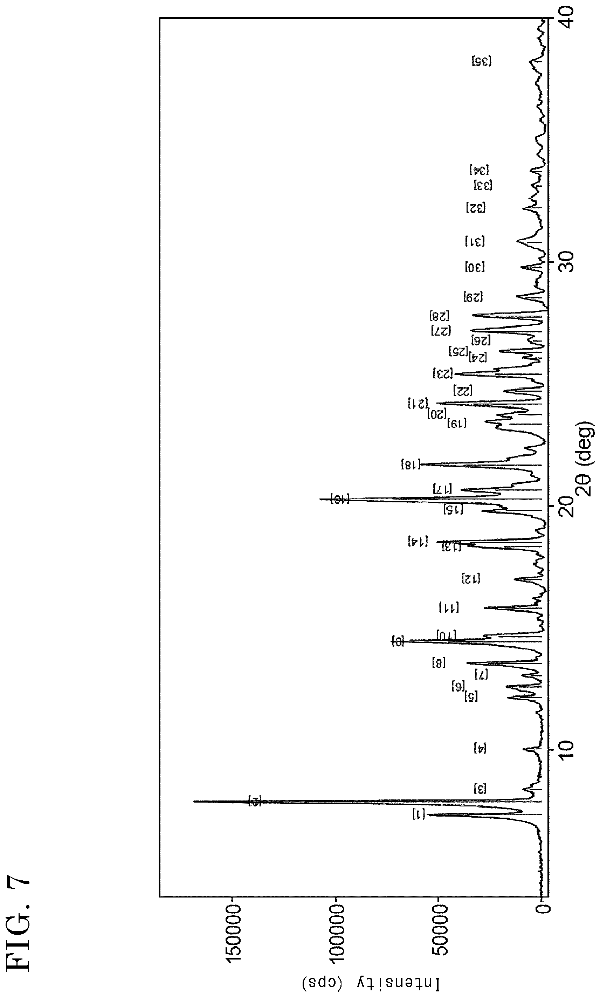

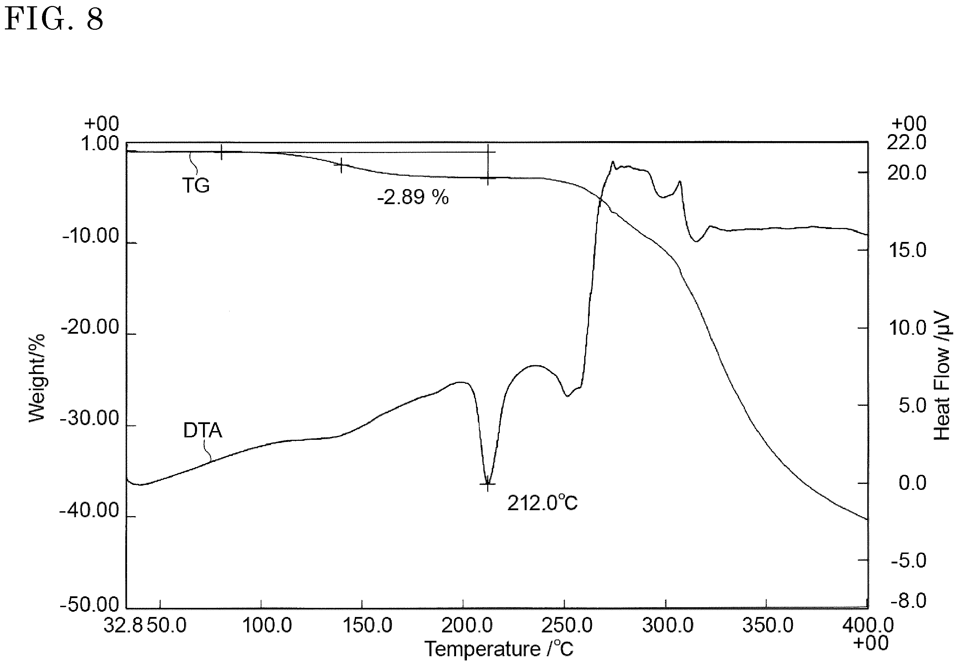

The refined product of the compound 9 (37.0 kg) obtained in step 6 and DMF (88.3 kg) were added to a stirring tank. The temperature of the mixed solution was raised to 50 to 60°C to dissolve the compound 9 in DMF. At 45 to 60°C, acetonitrile (218 kg) was added to the solution. Then, at 45 to 60°C, a solution of p-toluenesulfonic acid hydrate (pTsOH-H2O, 12.8 kg) dissolved in acetonitrile (116 kg) was added dropwise. Furthermore, at 45 to 60°C, acetonitrile (175 kg) was added. Then, the slurry was stirred at a temperature of 50 to 60°C for 5 hours or longer, and then filtered to collect the insoluble material, and the collected insoluble material (cake) was washed with a mixed solution of DMF (8.7 kg) and acetonitrile (50.9 kg), and then acetonitrile (87.2 kg). Then, the collected insoluble material was dried under reduced pressure while maintaining the temperature outside the vessel at 60°C or lower, to obtain a crude product of the compound 9 as 39.0 kg of a powder (yield: 92%). The results of X-ray powder crystal analysis of the obtained powder are shown in FIG. 7. In addition, the results of thermophysical property measurement by TG-DTA of the obtained powder are shown in FIG. 8. The TG-DTA shown in FIG. 8 was measured by TG-8120 (manufactured by Rigaku Corporation) (cell: alumina (open), gas: nitrogen, heating rate: 10.0°C/min, hold temperature: 400°C, hold time: 0 min). The content of residual solvent in the obtained powder was 22659 ppm for acetonitrile and 5824 ppm for DMF.

## Claims

1. A production method for a p-toluenesulfonate of a compound represented by the following formula (I): 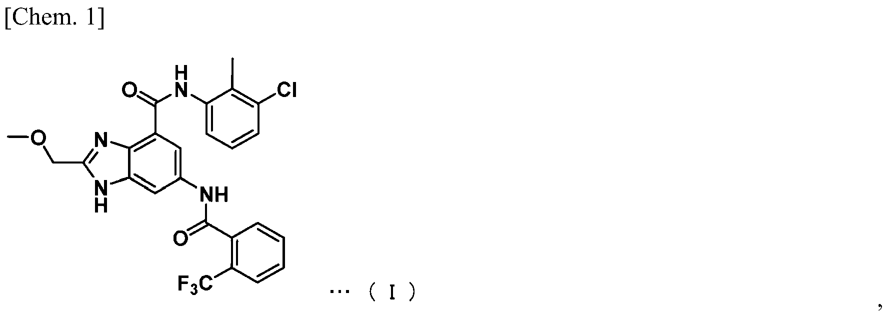 including a step of converting an N,N-dimethylacetamide solvate of the compound represented by the formula (I) into the p-toluenesulfonate of the compound represented by the formula (I).

2. The production method according to claim 1, further including a step of converting a compound represented by the following formula (II): 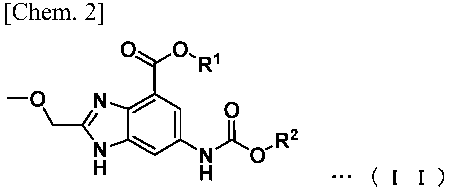 (wherein R¹ represents a protecting group for a carboxyl group or H, and R² represents a group that combines with -O-C(=O)- to form a protecting group for an aniline amino group) into an N,N-dimethylacetamide solvate of a compound represented by the following formula (I):

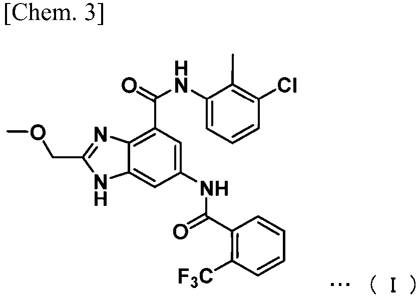

3. The production method according to claim 2, wherein the step of converting the compound represented by the formula (II) into the compound represented by the formula (I) includes hydrolyzing the compound represented by the formula (II) in the presence of a base to obtain a compound represented by the following formula (III):

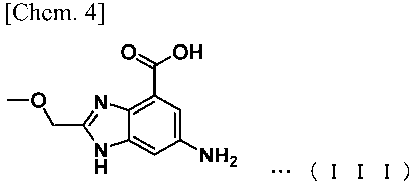

4. The production method according to claim 2, wherein R¹ and R² in the formula (II) each independently represent C₁-C₆ alkyl.

5. The production method according to claim 2, wherein R¹ and R² in the formula (II) are each methyl.

6. The production method according to any one of claims 2 to 5, further including a step of obtaining the compound represented by the formula (II), including bringing a compound represented by the following formula (IV): 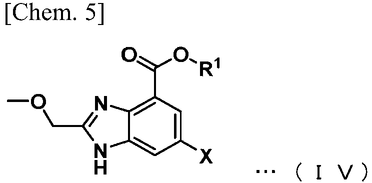
(wherein R¹ is the same as R¹ in the formula (II), and X represents Cl, Br, I, or OTf) or its protected product into contact with a compound represented by the following formula (V): 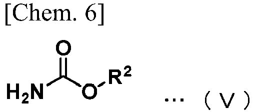
(wherein R² is the same as R² in the formula (II))
to obtain the compound represented by the formula (II) or its protected product.

7. The production method according to claim 6, wherein bringing the compound represented by the formula (IV) or its protected product into contact with the compound represented by the formula (V) in the step of obtaining the compound represented by the formula (II) is performed in the presence of a palladium catalyst.

8. The production method according to claim 6, wherein X in the formula (IV) is Br.

9. The production method according to claim 6, further including a step of bringing a compound represented by the following formula (VI): 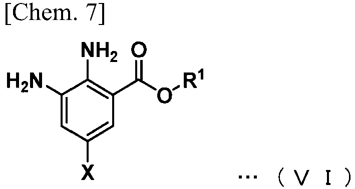 (wherein R¹ is the same as R¹ in the formula (II), and X is the same as X in the formula (IV)) into contact with methoxyacetic acid to obtain the compound represented by the formula (IV).

10. The production method according to claim 1, further including a step of purifying the N,N-dimethylacetamide solvate of the compound represented by the formula (I), by recrystallization.

11. An N,N-dimethylacetamide solvate of a compound represented by the following formula (I):

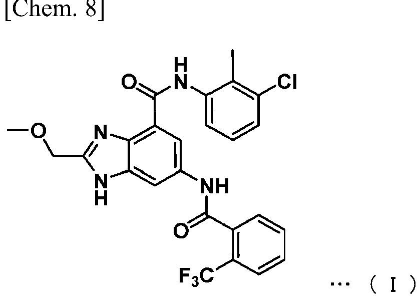

12. A composition containing an N,N-dimethylacetamide solvate of a compound represented by the following formula (I): 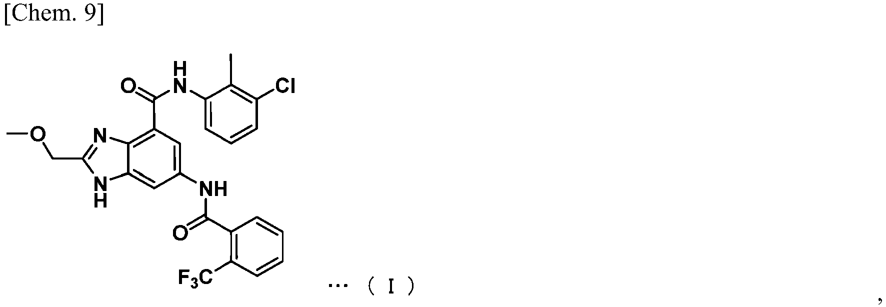
wherein a content of the N,N-dimethylacetamide solvate of the compound represented by the formula (I) is 95 mass% or more of the entire composition, and a content of a compound represented by the following formula (VII): 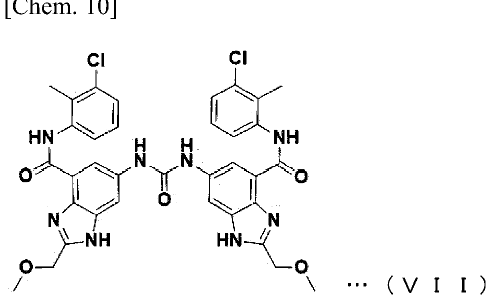
is 1800×10⁻⁴ mass% or less of the entire composition in terms of free form.

13. A composition containing a p-toluenesulfonate of a compound represented by the following formula (I): 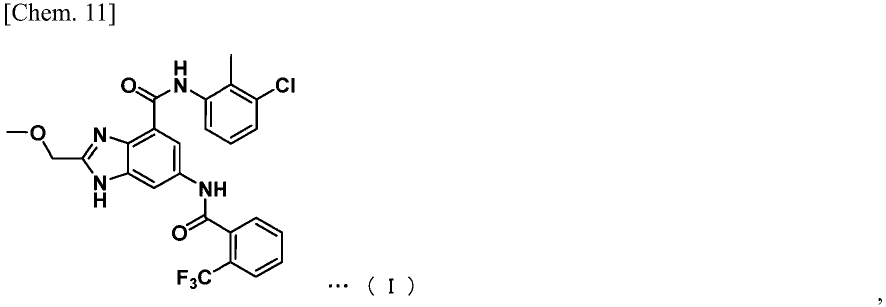 and
a compound represented by the following formula (VII): 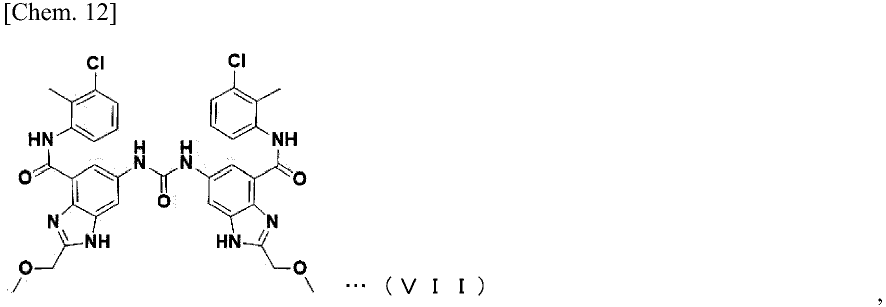
wherein a content of the p-toluenesulfonate of the compound represented by the formula (I) is 95 mass% or more of the entire composition, and a content of the compound represented by the formula (VII) is 100×10⁻⁴ mass% or less in terms of free form.
